# EUROPÄISCHE PATENTSCHRIFT

(11) **EP 0 946 483 B1**
(45) Veröffentlichungstag und Bekanntmachung des Hinweises auf die Patenterteilung: **04.09.2002**
(21) Anmeldenummer: 97952875.9
(22) Anmeldetag: 04.12.1997
(51) Int. Cl.: C07C 45/75, C07C 47/19, C07C 29/141, C07C 31/22

(54) **VERFAHREN ZUR HERSTELLUNG VON POLYALKOHOLEN**
METHOD FOR PRODUCING POLYALCOHOLS
PROCEDE POUR PRODUIRE DES POLYALCOOLS

(30) Priorität: 20.12.1996 DE 19653093
(43) Veröffentlichungstag der Anmeldung: 06.10.1999
(73) Patentinhaber: BASF AKTIENGESELLSCHAFT, 67056 Ludwigshafen (DE)
(72) Erfinder: KRATZ, Detlef, D-69121 Heidelberg (DE); STAMMER, Achim, D-67251 Freinsheim (DE); SCHULZ, Gerhard, D-67069 Ludwigshafen (DE); VOIT, Guido, D-69198 Schriesheim (DE)
(74) Vertreter: Isenbruck, Günter, Dr.
(86) Internationale Anmeldenummer: EP9706776
(87) Internationale Veröffentlichungsnummer: WO98028253

(56) Entgegenhaltungen:
- EP-A- 0 142 090
- DE-A- 1 952 738
- DE-A- 2 507 461

## Beschreibung

Die Erfindung betrifft ein Verfahren zur Herstellung von geminalen Polymethylolverbindungen durch Kondensation von Aldehyden mit Formaldehyd und Hydrierung der erhaltenen Polymethylolalkanale, ohne Koppelproduktion von Formiaten.

### Stand der Technik

### Verfahren unter Bildung eines Koppelproduktes

Trimethylolpropan (TMP) wird großtechnisch in der Regel nach dem sogenannten anorganischen Cannizzaro-Verfahren hergestellt. Dabei wird n-Butyraldehyd (n-BA) mit überschüssigem Formaldehyd (FA) in Gegenwart stöchiometrischer Mengen einer anorganischen Base wie NaOH oder Ca(OH)₂ zur Reaktion gebracht. Neben TMP entsteht durch Cannizzaro-Reaktion ein Äquivalent Natrium- oder Calciumformiat. Der Anfall eines anorganischen Salzes als Koppelprodukt ist in mehrfacher Hinsicht nachteilig. Erstens ist die Trennung des Salzes von TMP kompliziert und erfordert zusätzlichen Aufwand, zweitens muß das anorganische Salz - falls es nutzbringend verwertet werden soll - aufgearbeitet und gereinigt werden und drittens bedeutet der Anfall eines Koppelproduktes einen Verlust der stöchiometrisch eingesetzten Mengen an Natronlauge und Formaldehyd. Außerdem sind die Ausbeuten bei dieser anorganischen Cannizzaro-Reaktion auch in Bezug auf den Butyraldehyd unbefriedigend, da im Laufe der Reaktion hochsiedende Bestandteile gebildet werden, die nicht weiter verwertet werden können.

Ähnliche Probleme wie für TMP geschildert liegen bei der Herstellung anderer Triole wie Trimethylolethan (aus n-Propanal und Formaldehyd) oder Trimethylolbutan (aus n-Pentanal und Formaldehyd) vor. Wie TMP werden diese Triole unter Koppelproduktion eines anorganischen Salzes nach dem anorganischen Cannizzaro-Verfahren hergestellt. Das gleiche gilt für die Herstellung von Pentaerythrit aus Acetaldehyd und Formaldehyd. Trimethylolpropan, Trimethylolethan und Trimethylolbutan sind Polyole, die im Kunststoffsektor zur Herstellung von Lacken, Urethanen und Polyestern vielfache Verwendung finden. Pentaerythrit ist ein in der Lackindustrie häufig eingesetztes Ausgangsmaterial und wird außerdem zur Herstellung von Sprengstoff (Pentaerythrittetranitrat) verwendet.

Gemäß einem verbesserten Verfahren wird die Umsetzung von n-BA mit FA in Gegenwart eines tert. Amins in DE-A 1 952 738 beschrieben. Die verwendete Stöchiometrie n-BA/FA von ca. 1/6 und die Verwendung von überstöchiometrischen Mengen des Amins haben jedoch zur Folge, daß neben TMP stöchiometrische Mengen Trialkylammoniumformiat entstehen. Amin und gebildete Ameisensäure werden als organisches Salz abdestilliert und müssen somit verarbeitet und wiedergewonnen werden, um das Verfahren wirtschaftlich zu gestalten. Als Amine wurden Triethyl- und Trimethylamin verwendet.

Eine Möglichkeit den Anfall von organischem Salz (Trialkylammoniumformiat) zu vermeiden, ist in EP-A 0 142 090, EP-A 0 289 921 und in den deutschen Patentanmeldungen Aktenzeichen P 19542035.7 und P 19542036.5 beschrieben. Dort wird ebenfalls anstatt einer anorganischen Base die Verwendung stöchiometrischer Mengen eines Trialkylamins als Base beschrieben. Das als Koppelprodukt entstehende Trialkylammoniumformiat, wird im Laufe des Prozesses in Methylformiat umgewandelt. Der Vorteil dieses organischen Cannizzaro-Verfahrens gegenüber der anorganischen Variante liegt in der erhöhten Ausbeute. Nachteilig bleibt die Tatsache, daß auch hier ein Koppelprodukt (Methylformiat) entsteht und somit ein Äquivalent Formaldehyd mehr als notwendig in dem Verfahren verbraucht wird.

Ein Verfahren zur Herstellung von TMP ohne jegliches Koppelprodukt war somit aus wirtschaftlicher Sicht wünschenswert.

### Verfahren ohne Bildung eines Koppelproduktes

Gemäß den Angaben von DE-A 25 07 461 kann Dimethylolbutanal (DMB) durch Umsetzung von n-Butyraldehyd (n-BA) mit Formaldehyd (FA) in Gegenwart katalytischer Mengen eines tert. Amins über das Zwischenprodukt Monomethylolbutanal (MMB) hergestellt werden (Gleichung 1). Anschließende Hydrierung führt zu TMP (Gleichung 2).

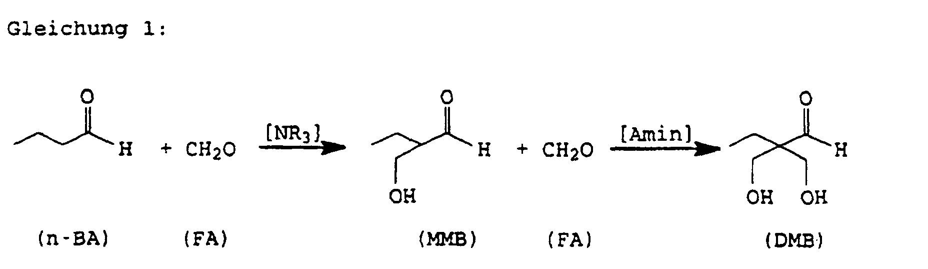

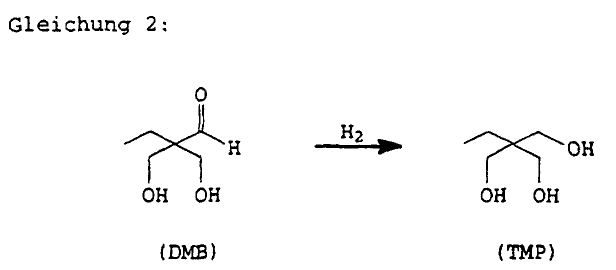

Die Hydrierung des aus n-BA und FA als Zwischenprodukt gebildeten Dimethylolbutanals (DMB) zu TMP wird ebenfalls in DE-A 25 07 461 beschrieben. Aus der gleichen Schrift ist bekannt, daß aus dem intermediär gebildeten Monomethylolbutanal (MMB) durch Abspaltung von Wasser als Nebenprodukt Ethylacrolein (EA) gebildet wird. Die Möglichkeit, EA über seine Rückreaktion in Gegenwart von Wasser zu verwerten (Gleichung 3), wird in Beispiel 5 erläutert.

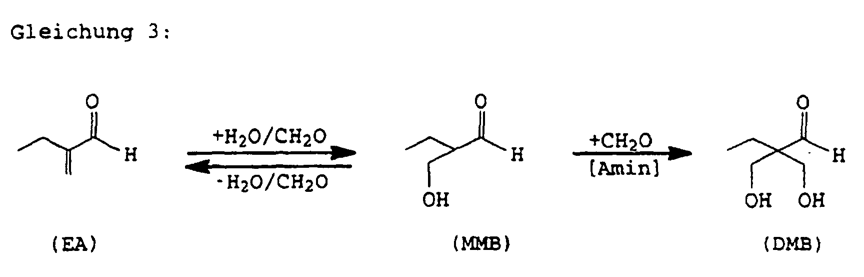

Die in den Beispielen erwähnten Ausbeuten zu DMB liegen bei ca. 85 %; nach Hydrierung werden TMP-Ausbeuten von 75 % (bez. n-BA) beschrieben. Eine Ausbeute bez. FA ist nicht angegeben.

Das Verfahren dieser Patentschrift hat den Nachteil, daß spezielle verzweigte tert. Amine (z.B. Dimethylaminoneopentanolamin), die nicht in technischen Mengen verfügbar sind, verwendet werden müssen. Mit industriell verfügbaren Aminen wie Triethylamin werden schlechtere Ausbeuten beobachtet (57 % TMP bezüglich n-BA). Das verzweigte Amin wird in dem beschriebenen Verfahren nicht in den Prozeß zurückgeführt und führt dadurch zu erhöhten Kosten. Ebenso wird unumgesetzter n-BA und FA nicht in den Prozeß zurückgeführt.

Eine weitere Variante ist in DE-A 27 02 582 beschrieben, bei der man Formaldehyd in mindestens 8-fachem Überschuß gegenüber dem Aldehyd zur Aldolisierung in Gegenwart tert. Amine in Kombination mit Alkali- oder Erdalkaliverbindungen bei -5 bis 0°C einsetzt. Das gebildete Aldolprodukt wird anschließend zu TMP hydriert. Als Basen werden lineare tert. Amine verwendet. In den meisten Beispielen werden zusätzlich anorganische Basen eingesetzt. Die Ausbeute bei alleiniger Verwendung von Triethylamin (Beispiel 5, n-BA/FA/NEt₃ = 1/10/0,18) beträgt nach Hydrierung 74,6 % TMP.

Dieses Verfahren beruht auf der Verwendung eines großen Überschusses Formaldehyd und tiefer Temperaturen. Hohe FA-Mengen führen jedoch vermehrt zur Bildung von FA-Addukten der im Reaktionsgemisch vorhandenen Alkoholkomponenten (Bildung von Acetalen und Halbacetalen des FA mit DMB und MMB). Dies führt wiederum zu einer unqünstigen Ausbeute bezogen auf eingesetzten FA. Die Verwendung anorganischer Reagenzien in der Aldolisierung führt zudem bei der späteren Aufarbeitung zu Problemen bei der Destillation von TMP. Die Einhaltung niedriger Temperaturen (-5 bis 0°C) erfordert zusätzlichen technischen Aufwand. Es werden insgesamt 18 mol-% tert. Amin eingesetzt, die nicht in den Prozeß rückgeführt werden.

Auch beim Verfahren der DE-A 28 13 201 ist wie in DE-A 27 02 582 ein hoher Überschuß an Formaldehyd kennzeichnend und eine akzeptable Ausbeute bez. n-Butyraldehyd wird nur auf Kosten des eingesetzten Formaldehyds erreicht, so daß auch dieses Verfahren unwirtschaftlich ist.

In DE-A 27 14 516 ist die Reaktion von Ethylacrolein (EA) mit Formaldehyd (FA) in einem Verhältnis EA/FA von 1:8 bis 1:30 in Gegenwart basischer Katalysatoren beschrieben. Die Ausbeute an DMB wird zu 90 % bez. EA angegeben. Bezüglich eingesetztem FA ergibt sich jedoch nur eine Ausbeute von 12 %.

Auch hier ist aufgrund der hohen FA-Mengen als auch des hohen Einsatzes von Trialkylaminkatalysator das Verfahren nicht wirtschaftlich.

Es bestand daher die Aufgabe ein Verfahren zu entwickeln, das ohne Bildung eines Koppelproduktes die Herstellung von Polymethylolverbindungen, z.B. Trimethylolpropan aus n-Butyraldehyd und Formaldehyd, in hohen Ausbeuten bezüglich n-Butyraldehyd und Formaldehyd, ermöglicht. Dieses Verfahren sollte gleichermaßen für die Herstellung anderer Polyalkohole aus den höheren und niederen homologen Alkanalen des n-Butyraldehyds geeignet sein.

Diese Aufgabe wurde gelöst mit einem Verfahren zur Herstellung von Hydroxymethylalkanalen der Formel I 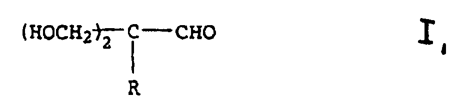 in der R eine weitere Methylolgruppe oder eine Alkylgruppe mit 1 bis 22 C-Atomen oder eine Aryl- oder Aralkylgruppe mit 6 bis 22 C-Atomen bedeutet, durch Kondensation von Aldehyden mit 2 bis 24 C-Atomen mit Formaldehyd unter Verwendung von tertiären Aminen als Katalysator, das dadurch gekennzeichnet ist, daß man die Kondensation stufenweise durchführt, wobei man
a) in einer ersten (Reaktions-)Stufe die Aldehyde mit 2 oder mehr C-Atomen mit der 2- bis 8-fachen molaren Menge Formaldehyd in Gegenwart eines tertiären Amins als Katalysator umsetzt,
b) in einer zweiten (Trenn-)Stufe entweder das Reaktionsgemisch in einen überwiegend die Verbindungen der Formel I enthaltenden Sumpf und einen aus überwiegend nicht umgesetzten oder teilumgesetzten Ausgangsmaterialien bestehenden Destillatstrom trennt, der in die erste Stufe zurückgeführt wird, oder das Reaktionsgemisch aus der ersten Stufe mittels einer Phasenscheidevorrichtung in eine wäßrige und eine organische Phase trennt und die organische Phase in die erste Stufe zurückführt und
c) in einer dritten (Nachreaktions-Destillations-)Stufe den Sumpf der zweiten Stufe oder die in der zweiten Stufe durch Phasenscheidung erhaltene wäßrige Phase einer katalytischen und/oder thermischen Behandlung unterwirft, dabei die unvollständig methylolierte Verbindung der Formel II 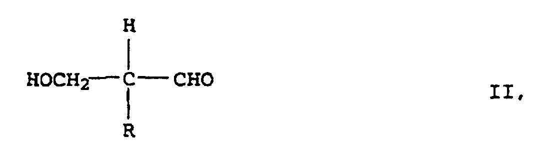 in die entsprechende Verbindung der Formel I und in die entsprechende Methylenverbindung der Formel III 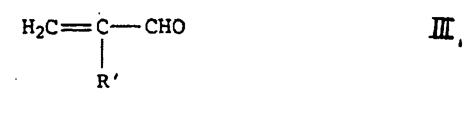 in der R' Wasserstoff ist oder die oben für R genannte Bedeutung hat, überführt, das so erhaltene Reaktionsgemisch destilliert, das Kopfprodukt dieser Destillation, das eine Verbindung der Formel III und nicht umgesetzten Formaldehyd enthält, in die erste Stufe zurückführt
und als Sumpfprodukt dieser Destillation die Verbindung der Formel I erhält.

Im Rahmen der folgenden Beschreibung des erfindungsgemäßen Verfahrens wird die "erste (Reaktions-)Stufe" synonym mit "Reaktions-)Stufe", "erste Stufe" oder "Stufe a)", die "zweite (Trenn-)Stufe" synonym mit "(Trenn-)Stufe" "zweite Stufe" oder "Stufe b)" und die "dritte (Nachreaktions-Destillations-)Stufe" synonym mit "(Nachreaktions-Destillations-)Stufe "dritte Stufe" oder "Stufe c)" bezeichnet.

Die Hydroxymethylalkanale der allgemeinen Formel I werden anschließend in an sich bekannter Weise zu den Polymethylolverbindungen der Formel IV

(HOCH₂)₃-C-R IV,

in der R die vorstehend bei Formel I gegebene Bedeutung aufweist, hydriert.

Nach einer bevorzugten Ausführungsform werden die rückgeführten Destillate oder die in der zweiten Stufe durch Phasenscheidung erhaltene, rückgeführte organische Phase, sofern sie wesentliche Mengen von Methylenverbindungen der Formel III enthalten, einer Vorreaktion mit Formaldehyd und tertiärem Amin unterworfen, ehe sie mit weiterem Aldehyd mit 2 oder mehr C-Atomen in Kontakt gebracht werden.

Nach einer weiteren bevorzugten Ausführungsform wird das Destillat oder die Destillatströme der zweiten Stufe einer thermischen Nachreaktion unterworfen und erneut destilliert und das erhaltene Destillat oder, gegebenenfalls nach mehrfacher Wiederholung dieser Operation, das letzte Destillat in die Stufe a) zurückgeführt.

Somit bestehen bevorzugte Ausführungsformen des vorliegenden Verfahrens vor allem in der Anwendung einer Vorreaktion des die Methylenverbindungen der Formel III enthaltenden Destillats mit Formaldehyd und/oder gegebenenfalls in der Durchführung einer katalytischen und/oder thermischen Nachreaktion der Destillate vor deren Rückführung in die erste Stufe und/oder gegebenenfalls in der Durchführung einer katalytischen und/oder thermischen Nachreaktion der in der oder den Destillationen entstandenen wäßrigen Destillationssümpfe. Eine alternative bevorzugte Ausführungsform besteht in der Rückführung der in der Trennstufe durch Phasenscheidung erhaltenen organischen Phase und deren Vorreaktion mit Formaldehyd und/oder gegebenenfalls in der Durchführung einer katalytischen und/oder thermischen Nachreaktion der in der Trennstufe durch Phasenscheidung erhaltenen wäßrigen Phase.

Das erfindungsgemäße Verfahren beinhaltet im besonderen eine Reaktionsführung, die die quantitative Umsetzung von eingesetztem Aldehyd und die quantitative Umsetzung von intermediär gebildeten Methylenverbindungen der Formel III zu den Verbindungen der Formel I ermöglicht. So führt die katalytische und/oder thermische Nachbehandlung in Stufe c) des erfindungsgemäßen Verfahrens und die Rückführung des dabei erhaltenen Destillats in Stufe a) zu einer besonders günstigen Verwertung der eingesetzten Ausgangsmaterialien. Als Folge hiervon werden in Stufe a) zum einen die eigentlichen Reaktionskomponenten Aldehyd und FA eingesetzt, zum anderen die aus den Stufen b) und c) stammenden, rückgeführten Ströme, die Aldehyd, Methylenverbindung der Formel III, Formaldehyd, Wasser und Aminkatalysator enthalten.

Überraschenderweise können diese Ströme quantitativ wieder in die Reaktion eingesetzt werden, ohne daß dabei unerwünschte Nebenkomponenten erzeugt werden. Die störungsfreie Rückführung dieser Ströme gelingt besonders gut, wenn man die Umsetzung so durchführt, daß diese Ströme zuerst in einen Vorreaktor mit frischem FA zur Reaktion gebracht werden und nachfolgend in Stufe a) die Reaktion des so erhaltenen Gemisches mit den weiteren Ausgangsverbindungen durchgeführt wird. Dieses Vorgehen der "gestaffelten Zugabe" der Reaktionskomponenten führt gegenüber dem Stand der Technik zu erheblich verbesserten Ausbeuten.

Ein wesentliches Merkmal des erfindungsgemäßen Verfahrens besteht in der Nachreaktion des Destillationssumpfes oder der Destillationssümpfe bzw. der in der Trennstufe durch Phasenscheidung erhaltenen wäßrigen Phase, die noch monomethylolierte bzw., im Falle der Acetaldehyds, mono- und dimethylolierte Zwischenverbindungen enthalten, die hierdurch vor der Hydrierung in die betreffende vollständig methylolierte Verbindung und die betreffende Verbindung der allgemeinen Formel III überführt werden. Dies geschieht entweder in einem getrennten Nachreaktor, gegebenenfalls unter weiterer Zugabe von tertiärem Amin, oder in einer Reaktionskolonne oder in einer Kombination von beidem.

Das neue Verfahren kann diskontinuierlich als auch kontinuierlich durchgeführt werden.

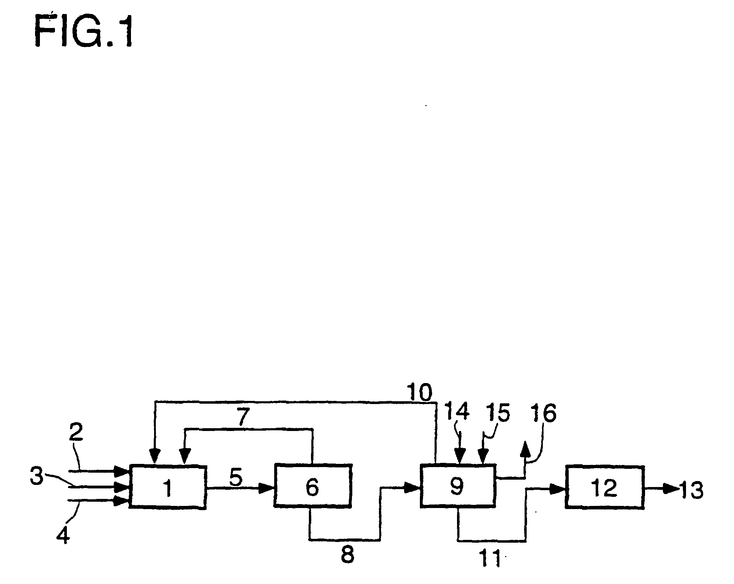

Zur Veranschaulichung des allgemeinen Verfahrensprinzips wird dieses im folgenden anhand der Zeichnung 1 für die Herstellung von TMP schematisch erläutert:

In der ersten (Reaktions-)Stufe 1, die aus einem einzelnen oder mehreren Reaktoren, vorzugsweise aus einem Rohrreaktor oder einer Rührkesselkaskade bestehen kann, werden die Ausgangsstoffe n-BA und FA, der vorzugsweise in Form einer wäßrigen Lösung eingebracht wird, sowie der tert.-Amin-Katalysator, welche über die Zuführungen 2, 3 bzw. 4 eingeleitet werden, zu einem Gemisch aus den Verbindungen DMB und MMB umgesetzt, das außerdem noch nicht umgesetzten n-BA, FA und EA sowie den Amin-Katalysator und gegebenenfalls Wasser als wesentliche Bestandteile enthält. Über die Leitung 5 wird dieses Gemisch der (Trenn-)Stufe, in diesem Falle der Destillationsvorrichtung 6 zugeführt, beispielsweise einer Kolonne oder einem Dünnschichtverdampfer, in der das Gemisch destillativ in leichter und schwerer flüchtige Bestandteile aufgetrennt wird. Durch die Einstellung geeigneter Destillationsbedingungen in 6 bildet sich eine Fraktion aus Leichtsiedern, die als wesentliche Komponenten nicht umgesetzten n-BA und FA, Wasser und einen Teil des Aminkatalysators, sowie EA, das bereits im Austrag aus der (Reaktions-)Stufe enthalten ist und zusätzlich durch Abspaltung von Wasser aus MMB unter den Destillationsbedingungen gebildet wird, enthält. Diese Leichtsiederfraktion wird über Leitung 7 nach ihrer Kondensation in einem Kühler (nicht eingezeichnet) wieder in die (Reaktions-)Stufe 1 zurückgeführt. Das schwerer flüchtige Sumpfprodukt der Destillation 6, das im wesentlichen aus DMB, MMB und einem Teil des Aminkatalysators zusammengesetzt ist, gelangt über Leitung 8 in die (Nachreaktions-Destillations-)Stufe 9, in die gewünschtenfalls über die Leitungen 14 und 15 zusätzlich frischer FA und Aminkatalysator eingetragen wird.

Die (Nachreaktions-Destillations-)Stufe 9 kann aus einem oder vorzugsweise mehreren Rührkesseln oder einem oder mehreren Rohrreaktoren bestehen, an den oder die sich eine Destillationsanlage, z.B. eine Kolonne oder ein Dünnschicht- oder Sambay-Verdampfer, anschließt. In Stufe 9 wird das restliche MMB in DMB umgewandelt und das erhaltene Reaktionsgemisch anschließend durch destillative Abtrennung der Niedrigsieder Wasser, FA, EA und Aminkatalysator konzentriert. Diese Leichtsieder werden über Leitung 10 in die (Reaktions-)Stufe 1 zurückgeführt, wohingegen das schwererflüchtige DMB über Leitung 11 dem Hydrierreaktor 12 zugeführt und darin auf an sich herkömmliche Weise mit Wasserstoff (Zuleitung nicht eingezeichnet) katalytisch zu TMP hydriert wird, das über Leitung 13 ausgetragen wird. Erforderlichenfalls kann der Austrag aus dem Hydrierreaktor noch einer Reindestillation unterworfen werden. Die über die Leitungen 7 und 10 rückgeführten Ströme aus nicht umgesetzten Ausgangsmaterialien, Aminkatalysator, Wasser und dem Zwischenprodukt EA werden in der (Reaktions-)Stufe 1 zusammen mit den frisch zugeführten Ausgangsmaterialien wieder wie beschrieben umgesetzt. Dabei wird die Zufuhr frischer Ausgangsstoffe und gegebenenfalls frischen Katalysators durch geeignete, an sich herkömmliche Regelvorrichtungen vorteilhaft so eingestellt, daß sich in 1 ein stationäres Gleichgewicht ausbildet. Gegebenenfalls in Form wäßriger FA-Lösung kontinuierlich zugeführtes Wasser kann über Leitung 16 ausgeschleust werden. Eine solche Wasserausschleusung ist für die erfolgreiche Durchführbarkeit des erfindungsgemäßen Verfahrens nicht zwingend, hat aber den Vorteil, daß das Volumen der im Hydrierreaktor 12 zu hydrierenden DMB-Lösung verringert wird. Das Wasser kann selbstverständlich auch nach dem Hydrierreaktor 12 destillativ abgetrennt werden. Da die über Leitung 7 und 10 rückgeführten Verbindungen in 1, entsprechend dem sich darin ausbildenden stationären Gleichgewicht umgesetzt werden, wobei praktisch keine Nebenprodukte entstehen, kommt es in der Gesamtbilanz des erfindungsgemäßen Verfahrens zu einem praktisch quantitativen Umsatz der eingesetzten Ausgangsmaterialien und zu einer Selektivität für die Bildung von TMP von über 90 %, bezogen auf Butyraldehyd.

Alternativ zur vorstehend beschriebenen Arbeitsweise kann im erfindungsgemäßen Verfahren vorteilhaft auch so vorgegangen werden, daß man das über Leitung 5 aus der (Reaktions-)Stufe 1 ausgetragene Reaktionsgemisch einem Phasenscheide-Apparat 6a statt einer der Destillationsanlage 6 zuführt und in dieser Phasenscheidevorrichtung den zweiphasigen Reaktionsaustrag in eine wäßrige und eine organische Phase trennt. Diese Arbeitsweise ist insbesondere dann vorteilhaft, wenn das Reaktionsgemisch aus Reaktionsstufe 1 in zwei flüssigen Phasen anfällt, was in der Regel der Fall ist. Liegt der Reaktionsaustrag aus 1 hingegen in homogener flüssiger Form vor, so können daraus, z.B. durch Wasserzusatz, zwei Phasen erzeugt werden. Da eine quantitative Trennung von organischer und wäßriger Phase in der Phasenscheidevorrichtung 6a der (Trenn-)Stufe für den Erfolg des erfindungsgemäßen Verfahrens nicht zwingend ist, werden an die Art des zu verwendenden Phasenscheide-Apparates keine besonderen technischen Anforderungen gestellt, d.h. es können üblicherweise für Flüssig-Flüssig-Trennungen angewandte Phasenscheidevorrichtungen, wie sie in Ullmanns Encyklopädie der technischen Chemie, 4. Auflage, Band 2, S. 560 bis 565, Verlag Chemie, Weinheim 1972 beschrieben sind, z.B. Absetzbehälter, Zentrifugen oder Kolonnen mit Einbauten zur Verbesserung der Phasentrennung, wie Siebboden- oder Füllkörperkolonnen aber auch Kolonnen mit rotierenden Einbauten verwendet werden.

Die bei der Phasenscheidung erhaltene organische Phase, die als wesentliche Komponenten EA, nicht umgesetzten Butyraldehyd und geringere Mengen an Aminkatalysator, FA, MMB, Wasser und DMB enthält, wird wieder in die (Reaktions-)Stufe 1 zurückgeführt und die wäßrige Phase, die als wesentliche Komponenten DMB, geringere Mengen MMB sowie FA und Aminkatalysator enthält, wird der (Nachreaktions-Destillations-)Stufe 9 zugeführt. Daraus ergibt sich, daß im Falle der Verwendung einer Phasenscheidevorrichtung 6a anstelle einer Destillationsapparatur 6 die bei der Phasenscheidung erhaltene organische Phase auf analoge Weise wie die Leichtsieder und die wäßrige Phase auf analoge Weise wie das hochsiedende Sumpfprodukt im Falle der Anwendung einer Destillationsvorrichtung 6 im weiteren Verfahrensgang weiterverarbeitet werden.

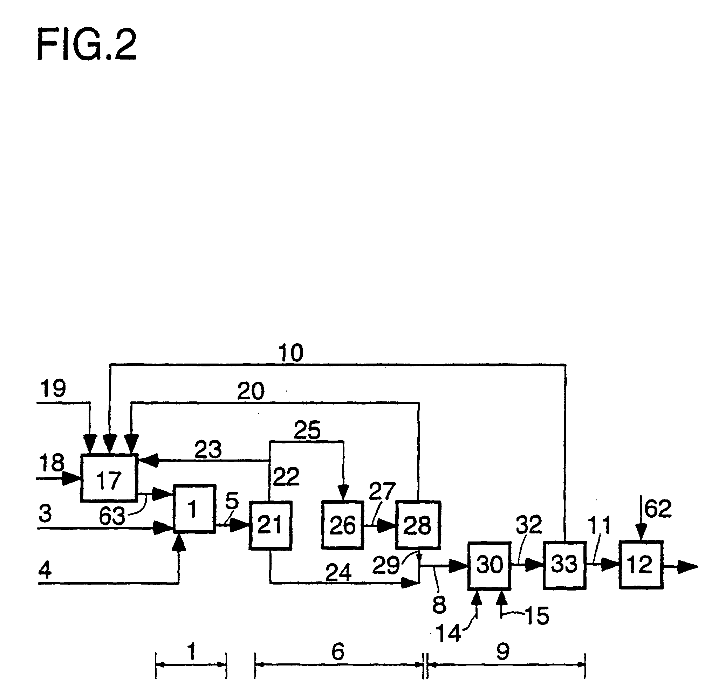

Im einzelnen geht man bei der Durchführung des erfindungsgemäßen Verfahrens vorteilhaft so vor, wie dies unter Einbeziehung bevorzugter und/oder alternativer Ausführungsformen anhand von Zeichnung 2 beispielhaft für die Herstellung von TMP aus n-BA und FA schematisch erläutert wird. Soweit die Vorrichtungen in Zeichnung 2 den Vorrichtungen in Zeichnung 1 direkt entsprechen, werden hierfür die gleichen Bezugszeichen verwendet.

In dem vorgeschalteten Reaktor 17, für die Zwecke dieser Anmeldung auch einfach als "Vorreaktor" bezeichnet, dem über die Leitungen 18 und 19, frischer FA, vorzugsweise in Form einer wäßrigen Lösung und soweit für die Einstellung gleichbleibender Betriebsbedingungen während des Dauerbetriebs des Verfahrens erforderlich, frischer tert.-Amin-Katalysator zudosiert wird, werden die über die Leitungen 20 und/oder 23 aus der (Trenn-)Stufe 6, gemäß Zeichnung 1 rückgeführten Leichtsieder, die nicht umgesetzten n-BA und FA, sowie gebildetes EA, außerdem Wasser und Aminkatalysator als wesentliche Bestandteile enthalten, sowie über Leitung 10 aus der (Nachreaktions-Destillations-)Stufe 9, gemäß Zeichnung 1, rückgeführtes FA/Wasser-Gemisch, das gegebenenfalls ebenfalls noch Aminkatalysator enthält, umgesetzt.

Im Vorreaktor 17 können die Edukte z.B. in folgenden Molverhältnissen vorliegen: Das Molverhältnis n-BA:EA kann je nach eingestelltem n-BA-Umsatz oder dem in den Destillationsvorrichtungen 21 und 28 eingestellten Rücklaufverhältnis stark schwanken und beträgt zwischen 1:1000 und 1000:1; das Molverhältnis (n-BA + EA)/FA beträgt im allgemeinen 1:0,01 bis 1:50, bevorzugt 1:2 bis 1:20 und die Menge an tertiärem Amin wird in der Regel so gewählt, daß der pH-Wert der Reaktionsmischung 5 bis 12, bevorzugt 6 bis 11, beträgt.

Die Gesamtkonzentration der Lösung der organischen Bestandteile in Wasser beträgt im Vorreaktor 17 im allgemeinen 5 bis 60 Gew.-%, bevorzugt 10 bis 45 Gew.-%. Die Reaktion wird im allgemeinen bei einer Temperatur von 5 bis 100°C, bevorzugt von 15 bis 80°C durchgeführt und die Verweilzeit wird - in Abhängigkeit von der Temperatur - im allgemeinen auf 0,25 bis 12 Stunden eingestellt.

Der Austrag aus dem Vorreaktor 17, der als wesentliche Bestandteile nicht umgesetzten FA und n-BA, EA, MMB und DMB sowie den Aminkatalysator und Wasser enthält, wird über Leitung 63, der (Reaktions-)Stufe 1 zugeführt, die ein Rührkessel, ein Rohrreaktor oder vorzugsweise eine Rührkesselkaskade sein kann. In die (Reaktions-)Stufe 1 wird über Leitung 3 frischer n-BA und erforderlichenfalls zur Einregulierung des stationären Gleichgewichts oder des pH-Wertes in 1, zusätzlich Aminkatalysator über Leitung 4 zudosiert. Im Falle der Verwendung einer Rührkesselkaskade kann diese Dosierung so erfolgen, daß entweder nur der erste Rührkessel der Kaskade, ein Teil der Rührkessel oder alle Rührkessel mit n-BA bzw. dem tert. Amin über die Leitungen 3 bzw. 4 beschickt werden. Die Zugabe des tert. Amins erfolgt in der Regel so, daß der pH-Wert wiederum 5 bis 12, bevorzugt 6 bis 11 beträgt. Bei Verwendung eines Rohrreaktors kann an verschiedenen Stellen des Rohres Aminkatalysator nachdosiert werden, um über die Länge des Reaktionsrohres den gewünschten pH-Bereich einzuregulieren.

Das Molverhältnis von frisch zugesetztem n-BA in 1 zu der im Vorreaktor 17 hinzugefügten Menge FA beträgt zweckmäßigerweise zwischen 1:2 und 1:5, bevorzugt 1:2 bis 1:3,5. Der noch im Überschuß im Austrag aus dem Vorreaktor 17 vorhandene FA reagiert in (Reaktions-)Stufe 1 mit n-BA im wesentlichen zu DMB. Die Menge an in den Vorreaktor 17 und in die (Reaktions-)Stufe 1 eingespeistem tert.-Aminkatalysator bezüglich des in die (Reaktions-)Stufe 1 eingetragenen n-BA beträgt in der Regel 0,001 bis 0,2, bevorzugt 0,01 bis 0,07 Äquivalente, d.h. das Amin wird in katalytischen Mengen eingesetzt. Die Gesamtkonzentration der organischen Bestandteile in der wäßrigen Reaktionsmischung beträgt im allgemeinen 5 bis 60 Gew.-%, bevorzugt 10 bis 45 Gew.-%. Die Reaktion wird im allgemeinen bei einer Temperatur von 5 bis 100°C, bevorzugt 15 bis 80°C durchgeführt. Die Verweilzeit wird - in Abhängigkeit von der Temperatur - in der Regel auf 0,25 bis 12 Stunden eingestellt.

Der Austrag aus (Reaktions-)Stufe 1, der im wesentlichen DMB, geringere Mengen MMB und EA, überschüssigen FA, geringe Mengen nicht umgesetzten n-BA, Wasser und Amin-Katalysator enthält, wird über Leitung 5 der (Trenn-)Stufe 6 zugeführt, die wie bei der Erläuterung von Zeichnung 1 geschildert konzipiert sein kann oder alternativ, wie im folgenden geschildert ausgestaltet sein kann, wobei die Gesamtheit der von der Klammer mit dem Bezugszeichen 6 in Zeichnung 2 umfaßten Apparaturen und Reaktoren, definitionsgemäß als zur (Trenn-)Stufe b), entsprechend (Trenn-)Stufe 6 gemäß Zeichnung 1, gehörig zu betrachten sind.

Der Austrag aus (Reaktions-)Stufe 1 wird bei Eintritt in die (Trenn-)Stufe 6 zunächst über Leitung 5 der Destillationsvorrichtung 21 aufgegeben, die z.B. eine Kolonne, ein Dünnschichtoder Sambay-Verdampfer sein kann, worin der Austrag aus 1 destillativ in Leichtsieder, die über Kopf oder aus dem oberen Teil der Destillationsvorrichtung 21 ausgetragen werden, und Hochsieder, die als Sumpfprodukt anfallen und aus dem unteren Teil der Destillationsvorrichtung 21 abgezogen werden, aufgetrennt. Die Destillation wird auf an sich herkömmliche Weise im allgemeinen bei 50 bis 200°C, vorzugsweise bei 90 bis 160°C und bei einem Druck von im allgemeinen 0,1 mbar bis 10 bar, vorzugsweise von 0,5 bis 5 bar, insbesondere bei Atmosphärendruck,durchgeführt. Die über Kopf oder dem oberen Teil der Destillationsvorrichtung 21 über Leitung 22 abgezogenen Leichtsieder enthalten als wesentliche Komponenten nicht umgesetzten n-BA und FA, EA, sowie Wasser und Aminkatalysator, wohingegen die aus dem Sumpf oder unteren Teil der Destillationsvorrichtung 21 über Leitung 24 abgeführten Hochsieder als wesentliche Komponenten DMB, eine geringere Menge MMB sowie Restmengen an Wasser, FA und Amin-Katalysator enthalten. Der Gehalt von DMB im aus 21 ausgetragenen hochsiedenden Produkt beträgt im allgemeinen 5 bis 90 Gew.-%.

Das in der Destillationsvorrichtung 21 erhaltene hochsiedende Gemisch wird über die Leitungen 24 und 8 der (Nachreaktions-Destillations-)Stufe 9 zugeführt, wohingegen das Leichtsiedergemisch vollständig oder als Teilstrom über die Leitungen 22 und 23 in den Vorreaktor 17 zurückgeführt werden kann und/oder über die Leitungen 22 und 25 nach Kondensation dem Reaktor 26, der z.B. ein Rührkessel, eine Rührkesselkaskade oder ein Rohrreaktor sein kann, zugeführt werden kann, wo es einer Nachreaktion bei im allgemeinen 10 bis 100°C, vorzugsweise 15 bis 80°C, für eine Zeitdauer von im allgemeinen 0,25 bis 12 Stunden unterworfen wird, bei der der im Destillat befindliche Amin-Katalysator die Umsetzung der im Destillat befindlichen Komponenten n-BA, EA und FA zu DMB und MMB bewirkt. Der Austrag aus dem Nachbehandlungsreaktor 26 wird über Leitung 27 in die Destillationsvorrichtung 28 geleitet. in der analog zur Destillation in Destillationsvorrichtung 21 wieder eine destillative Trennung in Leichtsieder und Hochsieder erfolgt, wobei die Leichtsieder über Leitung 20 wieder in den Vorreaktor 17 zurückgeführt und die Hochsieder über die Leitungen 29 und 8 in die (Nachreaktions-Destillations-)Stufe 9 geleitet werden.

Die voranstehend geschilderte Sequenz aus Nachreaktion/Destillation des Leichtsiederdestillats aus Destillationsvorrichtung 21 kann im Rahmen der (Trenn-)Stufe b), entsprechend der (Trenn-) Stufe 6 gemäß Zeichnungen, gewünschtenfalls mehrfach wiederholt werden. Diese Nachreaktion/Destillation-Sequenz im Rahmen der (Trenn-)Stufe 6 kann eine Alternative oder Ergänzung zur direkten Rückführung der Leichtsieder aus Destillationsvorrichtung 21 über die Leitungen 22 und 23 in den Vorreaktor 17 sein, die je nach Art der herzustellenden Polymethylolverbindung gleichwertig oder von Vorteil bezüglich einer direkten Rückführung sein kann. Die Rückführung der Leichtsieder aus (Trenn-)Stufe 6 in den Vorreaktor 17 ist eine vorteilhafte Ausführungsform des erfindungsgemäßen Verfahrens - selbstverständlich können die aus der (Trenn-)Stufe 6 rückgeführten Leichtsiederströme, vollständig oder als Teilströme, auch direkt in die (Reaktions-)Stufe a), entsprechend (Reaktions-)Stufe 1 in den Zeichnungen, zurückgeführt werden.

Je nachdem welche der vorstehend geschilderten Ausgestaltungen des erfindungsgemäßen Verfahrens angewandt wird, werden entweder die Hochsieder aus der Destillationsvorrichtung 21 über die Leitungen 24 und 8 oder die aus den Destillationsvorrichtungen 21 und 28 stammenden, über die Leitungen 24 und 29 vereinigten Hochsieder über Leitung 8 dem Nachbehandlungsreaktor 30 der (Nachreaktions-Destillations-)Stufe c), entsprechend der (Nachreaktions-Destillations-)Stufe 9 in den Zeichnungen, zwecks Vervollständigung der Umsetzung des im Hochsiedergemisch enthaltenden MMB mit FA zu DMB, zugeführt.

Wird anstelle der Destillationsvorrichtung 21 bei der anhand von Zeichnung 2 erläuterten Verfahrensausgestaltung eine Phasenscheidevorrichtung 21a verwendet, so kann mit der bei der Phasenscheidung erhaltenen organischen Phase, deren chemische Zusammensetzung in etwa der bei der Erläuterung von Zeichnung 1 beschriebenen Zusammensetzung entspricht, im weiteren Verfahrensgang auf analoge Weise wie mit den bei Verwendung einer Destillationsvorrichtung 21 erhaltenen Leichtsiedern verfahren werden und die bei der Phasenscheidung in 21a erhaltene wäßrige Phase kann im weiteren Verfahrensgang auf analoge Weise wie das bei Verwendung einer Destillationsvorrichtung 21 erhaltene hochsiedende Sumpfprodukt weiterbehandelt werden.

Im Nachbehandlungsreaktor 30, der z.B. ein Rührkessel oder vorzugsweise eine Rührkesselkaskade oder ein Rohrreaktor sein kann, wird das Hochsiedergemisch für eine Zeitdauer von im allgemeinen 0,1 bis 5 Stunden, vorzugsweise von 0,5 bis 3 Stunden, auf im allgemeinen 30 bis 200°C, vorzugsweise 40 bis 150°C erhitzt. Dabei bewirkt der im Hochsiedergemisch immer noch in katalytisch wirksamen Mengen enthaltene Amin-Katalysator bei der eingestellten Nachreaktionstemperatur die praktisch vollständige Umsetzung von MMB mit noch im Hochsiedergemisch vorhandenem FA oder gegebenenfalls über Leitung 15 zusätzlich eingetragenen FA zu DMB. Teilweise liegt der FA im Hochsiedergemisch auch in Form von Halbacetalen an MMB und DMB gebunden vor, aus denen er im Zuge dieser katalytischen und/oder thermischen Nachbehandlung freigesetzt wird, wodurch er zur Umwandlung von MMB zu DMB wieder zur Verfügung steht.

Die vorstehende Nachbehandlung und Nachreaktion der Hochsieder im Reaktor 30 kann beschleunigt und gewünschtenfalls unter milderen Bedingungen ausgeführt werden, wenn das Hochsiedergemisch unter Zugabe einer Base über Leitung 14, vorzugsweise des bei der Aldolisierung verwendeten tertiären Amin-Katalysators auf einen pH-Wert von 5 bis 12, bevorzugt 6 bis 11 gebracht wird und dann im vorstehend genannten Temperaturbereich behandelt wird. Anstatt eines niedermolekularen tertiären Amins können hierzu auch polymere tert. Amine, beispielsweise basische Ionenaustauscher, eingesetzt werden.

Der Austrag aus dem Nachreaktions-Reaktor 30 wird über Leitung 32 in eine Destillationsvorrichtung 33, beispielsweise einen Dünnschicht- oder Sambay-Verdampfer überführt und dort destillativ in hochsiedendes DMB und Leichtsieder, die als wesentliche Komponenten FA, Wasser, Amin-Katalysator und gegebenenfalls neu gebildetes EA enthalten, getrennt, wobei die Leichtsieder über Kopf oder aus dem oberen Teil der Destillationsvorrichtung abgezogen und über Leitung 10 z.B. in den Vorreaktor 17 und/oder gewünschtenfalls direkt in die (Reaktions-)Stufe 1 zurückgeführt werden können, wobei das hochsiedende DMB aus dem Sumpf oder unteren Teil der Destillationsvorrichtung 33 über Leitung 11 in den über Leitung 62 mit Wasserstoff versorgten Hydrierreaktor 12, eingeleitet wird. Falls gewünscht, kann die Destillation so gestaltet werden, daß ein Teilstrom, der im wesentlichen Wasser enthält, kondensiert und ausgeschleust wird.

Das nunmehr nahezu ausschließlich DMB als Reaktionsprodukt enthaltende hochsiedende Produkt aus 33, dessen Gehalt an DMB 20 bis 95 Gew.-%, bevorzugt 30 bis 75 Gew.-% beträgt, wird im Hydrierreaktor 12 katalytisch hydriert. Als Hydrierkatalysatoren eignen sich insbesondere Kupfer-haltige Trägerkatalysatoren wie sie z.B. in WO 95/32171 beschrieben sind. Ebenso sind Katalysatoren, wie sie in EP-A 44 444, EP-A 44 412 oder DE-A 19 57 592 beschrieben sind, geeignet. Die Hydrierung erfolgt zweckmäßigerweise kontinuierlich, z.B. in einem mit einer Katalysatorschüttung gefüllten Reaktorrohr, bei dem die Reaktionslösung über die Katalysatorschüttung z.B. in Rieselfahrweise oder im Bereich der Übergangsströmung, wie in DE-A 19 41 633 oder DE-A 20 40 501 beschrieben, geleitet wird. Es kann vorteilhaft sein, einen Teilstrom des Reaktionsaustrages, gegebenenfalls unter Kühlung, zurückzuführen und wieder über das Katalysatorfestbett zu leiten. Ebenso kann es vorteilhaft sein, die Hydrierung in mehreren hintereinander geschalteten Reaktoren durchzuführen, beispielsweise in 2 bis 4 Reaktoren, wobei in den einzelnen Reaktoren vor dem letzten Reaktor, die Hydrierreaktion nur bis zu einem Teilumsatz von z.B. 50 bis 98 % durchgeführt wird und erst im letzten Reaktor die Hydrierung vervollständigt wird. Dabei kann es zweckmäßig sein, den Hydrieraustrag aus dem vorangehenden Reaktor vor dessen Eintritt in den nachfolgenden Reaktor zu kühlen, beispielsweise mittels Kühlvorrichtungen oder durch Eindüsen kalter Gase, wie Wasserstoff oder Stickstoff oder Einleiten eines Teilstroms an kalter Reaktionslösung.

Die Hydriertemperatur liegt im allgemeinen zwischen 50 und 180°C, bevorzugt 90 und 140°C. Als Hydrierdruck werden im allgemeinen 10 bis 250 bar, bevorzugt 20 und 120 bar angewandt.

Die Hydrierung kann unter Zugabe eines inerten Lösungsmittels durchgeführt werden. Als Lösungsmittel sind cyclische Ether, wie THF oder Dioxan, als auch acyclische Ether einsetzbar, ebenso niedere Alkohole z.B. Methanol, Ethanol oder 2-Ethylhexanol.

Im übrigen können beliebige Hydriermethoden angewendet und Hydrierkatalysatoren eingesetzt werden, wie sie für die Hydrierung von Aldehyden üblich und in der Standardliteratur eingehend beschrieben sind.

Das so erhaltene Roh-TMP kann in an sich üblicher Weise durch Destillation gereinigt werden (nicht eingezeichnet).

Das erfindungsgemäße Verfahren kann mit oder ohne Zusatz von organischen Lösungsmitteln oder Lösungsvermittlern durchgeführt werden. Der Zusatz von Lösungsmitteln oder Lösungsvermittlern kann sich insbesondere bei der Verwendung langkettiger Aldehyde als Ausgangsmaterialien als vorteilhaft erweisen. Durch den Einsatz von Lösungsmitteln die geeignete, niedrigsiedende azeotrope Gemische mit den leichtsiedenden Verbindungen bei den einzelnen Destillationen des erfindungsgemäßen Verfahrens bilden, kann gegebenenfalls der Energieaufwand bei diesen Destillationen erniedrigt und/oder die destillative Abtrennung der Leichtsieder von den hochsiedenden Verbindungen erleichtert werden.

Als Lösungsmittel sind z.B. cyclische und acyclische Ether wie THF, Dioxan, Methyl-tert.butylether oder Alkohole wie Methanol, Ethanol oder 2-Ethylhexanol geeignet.

Die im Vorreaktor, der (Reaktions-)Stufe, der (Trenn-)Stufe und der (Nachreaktions-Destillations-)Stufe vorstehend im einzelnen beschriebenen Umsetzungen können bei einem Druck von im allgemeinen 1 bis 30 bar, vorzugsweise 1 bis 15 bar, besonders bevorzugt 1 bis 5 bar, zweckmäßigerweise unter dem Eigendruck des betreffenden Reaktionssystems, durchgeführt werden.

Das neue Verfahren ist auf praktisch alle Alkanale mit einer Methylengruppe in α-Stellung zur Carbonylgruppe anwendbar. Es können aliphatische Aldehyde mit 2 bis 24 C-Atomen als Ausgangsmaterialien verwendet werden, die geradkettig oder verzweigt sein oder auch alicyclische Gruppen enthalten können. Ebenso können araliphatische Aldehyde als Ausgangsstoffe eingesetzt werden, vorausgesetzt daß sie eine Methylengruppe in α-Stellung zur Carbonylgruppe enthalten. Im allgemeinen werden Aralkylaldehyde mit 8 bis 24 C-Atomen, vorzugsweise mit 8 bis 12 C-Atomen als Ausgangsmaterialien verwendet, beispielsweise Phenylacetaldehyd. Bevorzugt werden aliphatische Aldehyde mit 2 bis 12 C-Atomen, beispielsweise 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende -n-pentanale, -n-hexanale, -n-heptanale; 4-Ethyl-, 4-n-Propyl-, 4-Isopropyl-, 4-n-Butyl-, 4-Isobutyl-, 4-sek.-Butyl-, 4-tert.-Butyl-pentanale, -n-hexanale, -n-heptanale; 5-Ethyl-, 5-n-Propyl-, 5-Isopropyl-, 5-n-Butyl-, 5-Isobutyl-, 5-sek.-Butyl-, 5-tert. -Butyl-n-hexanale, -n-heptanale; 3-Methyl-hexanal, 3-Methyl-heptanal;4-Methyl-pentanal, 4-Methyl-heptanal, 5-Methyl-hexanal, 5-Methylheptanal; 3,3,5-Trimethyl-n-pentyl-, 3,3-Diethyl-pentyl-, 4,4-Diethylpentyl-, 3,3-Dimethyl-n-butyl-, 3,3-Dimethyl-n-pentyl-, 5,5-Dimethylheptyl-, 3,3-Dimethylheptyl-, 3,3,4-Trimethylpentyl, 3,4-Dimethylheptyl-, 3,5-Dimethylheptyl-, 4,4-Dimethylheptyl-, 3,3-Diethylhexyl-, 4,4-Dimethylhexyl-, 4,5-Dimethylhexyl-, 3,4-Dimethylhexyl-, 3,5-Dimethylhexyl-, 3,3-Dimethylhexyl-, 3,4-Diethylhexyl-, 3-Methyl-4-ethylpentyl, 3-Methyl-4-ethylhexyl-, 3,3,4-Trimethylpentyl-, 3,4,4-Trimethylpentyl-, 3,3,4-Trimethylhexyl-, 3,4,4-Trimethylhexyl-, 3,3,4,4-Tetramethylpentylaldehyd; insbesondere C₂- bis C₁₂-n-Alkanale.

Besonders bevorzugt werden Acetaldehyd zur Herstellung von Pentaerythrit, Propionaldehyd zur Herstellung von Trimethylolethan, n-BA zur Herstellung von TMP und n-Pentanal zur Herstellung von Trimethylolbutan als Ausgangsverbindungen eingesetzt.

Anstelle der Alkanale können auch Verbindungen der Formel IV, z.B.Acroleine, wie Acrylaldehyd (Acrolein); 2-Methylacrylaldehyd, 2-Ethylacrylaldehyd, 2-Propylacrylaldehyd, 2-Butylacrylaldehyd, 2-Pentylacrylaldehyd, 2-Isopropylacrylaldehyd, 2-Isobutylacrylaldehyd, 2-Hexylacrylaldehyd, 2-Heptylacrylaldehyd, 2-Dodecylacrylaldehyd, 2-Pentadecylacrylaldehyd, 2-Methoxyacrylaldehyd, 2-Ethoxyacrylaldehyd, 2-Propoxyacrylaldehyd oder 2-Butoxyacrylaldehyd, eingesetzt werden. Von diesen werden 2-Methylacrolein. 2-Ethylacrolein und 2-Propylacrolein bevorzugt. Gleichermaßen kann Acrolein vorteilhaft zur Gewinnung von Pentaerythrit eingesetzt werden.

Wie sich aus der eingangs angegebenen Gleichung 3 ergibt, ist bei Verwendung der Acroleine der allgemeinen Formel IV als Einsatzstoff ein Äquivalent weniger FA für die vollständige Umsetzung zu Verbindungen der Formel II erforderlich als beim Einsatz der entsprechenden gesättigten Aldehyde.

Als tert. Amine kommen an sich bezüglich ihrer Eignung für die Kondensation von Aldehyden mit Formaldehyd bekannte Amine, wie sie z.B. in DE-A 28 13 201 und DE-A 27 02 582 beschrieben sind, in Betracht. Besonders bevorzugt sind Tri-n-alkylamine, wie Triethylamin, Tri-n-propylamin, Tri-n-butylamin und insbesondere Trimethylamin.

Das erfindungsgemäße Verfahren zeichnet sich durch hohe Ausbeuten bezogen sowohl auf den Ausgangsaldehyd als auch auf den Formaldehyd aus und führt zu sehr geringen Verlusten an Aminkatalysator. Aufgrund des Arbeitens bei relativ niedrigen pH-Werten findet keine Cannizzaro-Reaktion statt, wodurch die Bildung von Formiatsalzen als Koppelprodukt vermieden wird.

### Beispiele

Beispiele 1 bis 4: Ansatzweise Arbeitsweise

### Beispiel 1

288 g n-Butyraldehyd (4 mol) wurden mit 3000 g 10%igem Formaldehyd (10 mol) und 26 g 45%igem Trimethylamin (0,2 mol) bei 25°C 5 h gerührt. In der Reaktionslösung fand man danach 0,7 mol n-BA (Umsatz n-BA = 82,5 %) und 0,5 mol EA (Selektivität zu EA = 15 %). Die Reaktionslösung wurde eingeengt, indem bei Normaldruck über einen Sambay-Verdampfer (140°C) bei einem Zulauf von 10 ml/h Leichtsieder und Wasser abdestilliert wurden. Man erhielt 1195 g Destillat und 2119 g Sumpf, der erneut am Sambay-Verdampfer eingeengt wurde. Es entstanden 795 g Destillat und 1324 g Sumpf. Dieser Sumpf wurde ein drittes mal am Sambay-Verdampfer eingeengt. Man erhielt 494 g Destillat und 830 g Sumpf. Die vereinigten Destillate (2484 g) wurden bei 30°C 5 h gerührt und wiederum durch Verdampfen am Sambay-Verdampfer (140°C) eingeengt. Daraus wurden 1435 g Sumpf und 1049 g Destillat erhalten. das wie in Beispiel 2 beschrieben, weiterverarbeitet wurde.

Die erhaltenen vereinigten Sümpfe (2265 g DMB-Lösung, pH 4,1), die kein n-BA oder EA enthielten, wurden einer kontinuierlichen Hydrierung unterworfen. Dazu wurde die Reaktionslösung zusammen mit Wasserstoff bei einem Druck von 30 bar in einen auf 90°C beheizten Rohrreaktor gepumpt, der mit 100 ml eines Kupfer auf SiO₂-Katalysators, der 25 Gew.-% Kupfer, berechnet als Cu und 75 Gew.-% SiO₂ enthielt und nach dem Verfahren gemäß WO95/32171 hergestellt worden war, gefüllt war. Die zu hydrierende Lösung wurde in Rieselfahrweise über die Katalysatorschüttung geleitet und ein Teil des noch unter Druck stehenden Reaktionsaustrages wurde in den Reaktor zurückgeführt (Kreislauffahrweise). Eine dem Zulauf entsprechende Menge wurde dem Reaktor kontinuierlich entnommen, entspannt und in einer Vorlage aufgefangen.

Der gesamte Hydrieraustrag wurde schließlich destilliert. Leichtsieder wurden mittels quantitativer Gaschromatographie (GC) erfaßt, Zwischensieder, TMP und höhere Kondensationsprodukte, umfassend Oligomere wie Di-Trimethylolpropan oder TMP-EA-TMP-Trimere, wurden ausgewogen:

| | MeOH | n-BuOH | Me-BuOH | Diol | TMP | höhere Kondensationsprodukte |
|---|---|---|---|---|---|---|
| Analysenmethode | GC | GC | GC | Auswaage | Auswaage | Auswaage |
| g | 25,6 | 2,9 | 2,5 | 8,3 | 375 | 8 |
| mol | 0,8 | 0,04 | 0,03 | 0,08 | 2,8 | 0,06 |
| (MeOH: Methanol; n-BuOH: n-Butanol; Me-BuOH: 2-Methylbutanol; Diol:3-Ethylpropandiol-1,3; TMP: Trimethylolpropan; höhere Kondensationsprodukte als TMP-Äquivalente bewertet) | | | | | | |

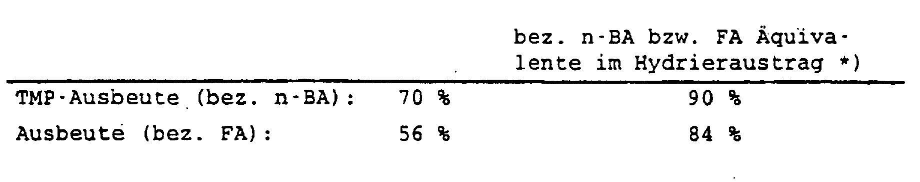

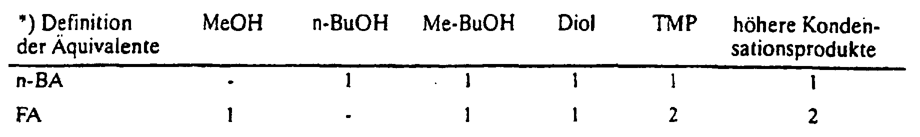

### Beispiel 2

Zu dem verbleibenden Destillat (1049 g) aus Beispiel 1 wurden 1951 g einer 9 mol FA enthaltenden wäßrigen Lösung und 26 g 45%iges Trimethylamin (0,2 mol) gegeben und die Mischung 3 h bei 30°C gerührt. Darauf wurden 288 g n-Butyraldehyd (4 mol) hinzugefügt und die Mischung weitere 4 h bei 30°C gerührt. Danach wurde wie in Beispiel 1 beschrieben destillativ aufgearbeitet. Man erhielt 873 g Destillat, das wie in Beispiel 3 beschrieben weiter verarbeitet wurde, und 2441 g Sumpf (DMB-Lösung), der nach Hydrierung und Destillation analog Beispiel 1 ein Produkt folgender Zusammensetzung ergab:

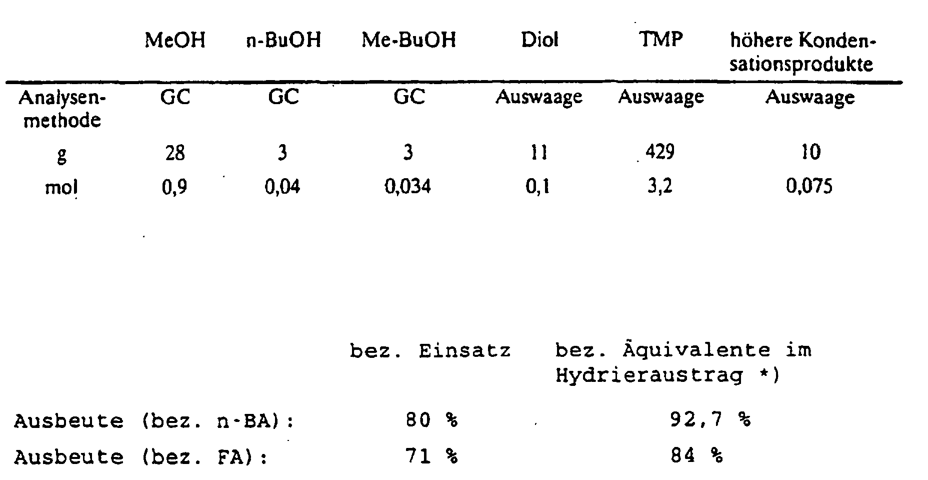

### Beispiel 3

Zu den 873 g Destillat aus Beispiel 2 wurden 2127 g einer 9 mol FA enthaltenden wäßrigen Lösung und 26 g 45%iges Trimethylamin (0,2 mol) gegeben und die Mischung wurde 3 h bei 30°C gerührt. Danach wurden 288 g n-Butyraldehyd (4 mol) hinzugefügt und weitere 4 h bei 30°C gerührt und anschließend wurde wie in Beispiel 1 angegeben destillativ aufgearbeitet. Man erhielt 1096 g Destillat, das wie in Beispiel 4 beschrieben weiter verarbeitet wurde und 2218 g DMB-Lösung die nach Hydrierung und Destillation folgende Produktzusammensetzung lieferte:

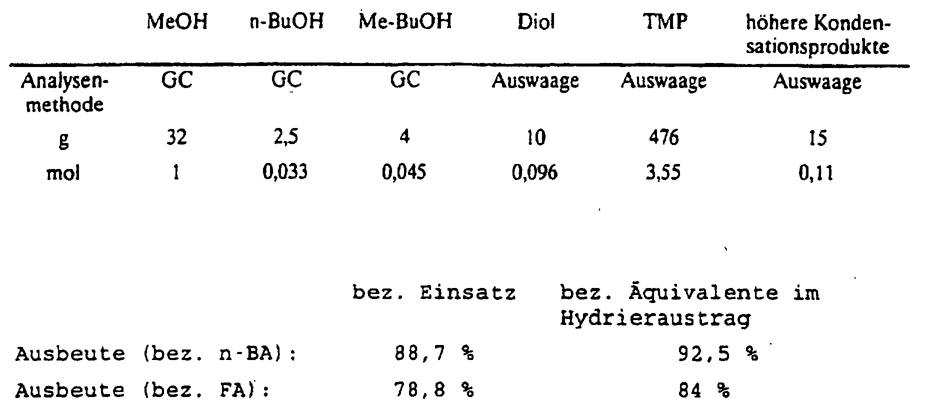

### Beispiel 4

Zu den 1096 g Destillat aus Beispiel 3 wurden zuerst 1904 einer 8,5 mol FA enthaltenden wäßrigen Lösung und 26 g 45%iges Trimethylamin (0,2 mol) gegeben und die Mischung 5 h bei 30°C gerührt. Danach wurden 288 g n-Butyraldehyd (4 mol) hinzugefügt und weitere 5 h bei 30°C gerührt. Nach destillativer Aufarbeitung wie in Beispiel 1 beschrieben, erhielt man 1150 g Destillat, das weiter verarbeitet werden kann. Die als Sumpf anfallende 2164 g DMB-Lösung ergab nach Hydrierung und Destillation folgende Mengen Produkt:

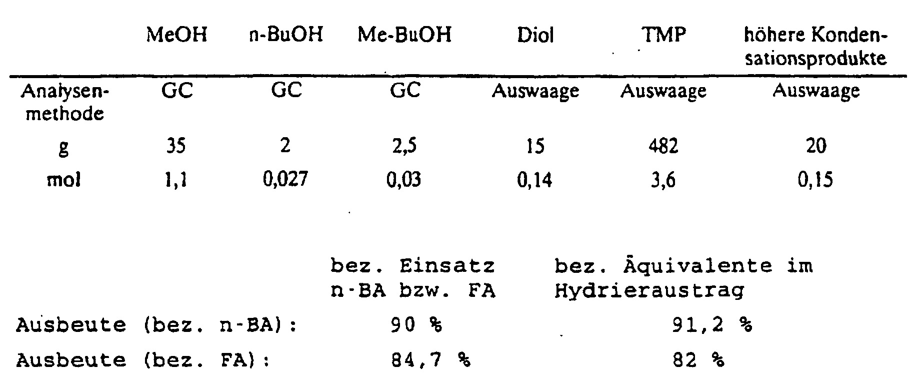

Kumuliert über Beispiele 1 bis 4 ergibt sich folgende Bilanz:

| | | |
|---|---|---|
| Einsatz n-BA | Einsatz FA | Einsatz NMe₃ (45 %ig) |
| 1152 g (16 mol) | 9558 g (36,5 mol) | 104 g (0,79 mol) |

im Destillat (zur Rückführung) verbleiben: 1,75 Äq. n-BA und 5,1 Äq. FA

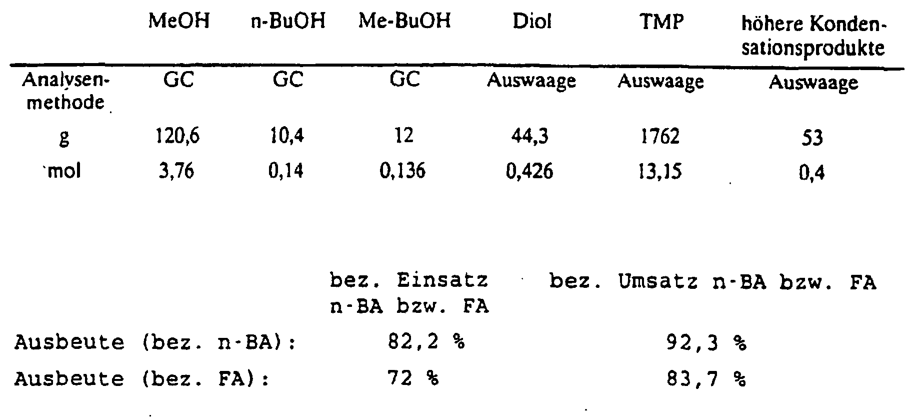

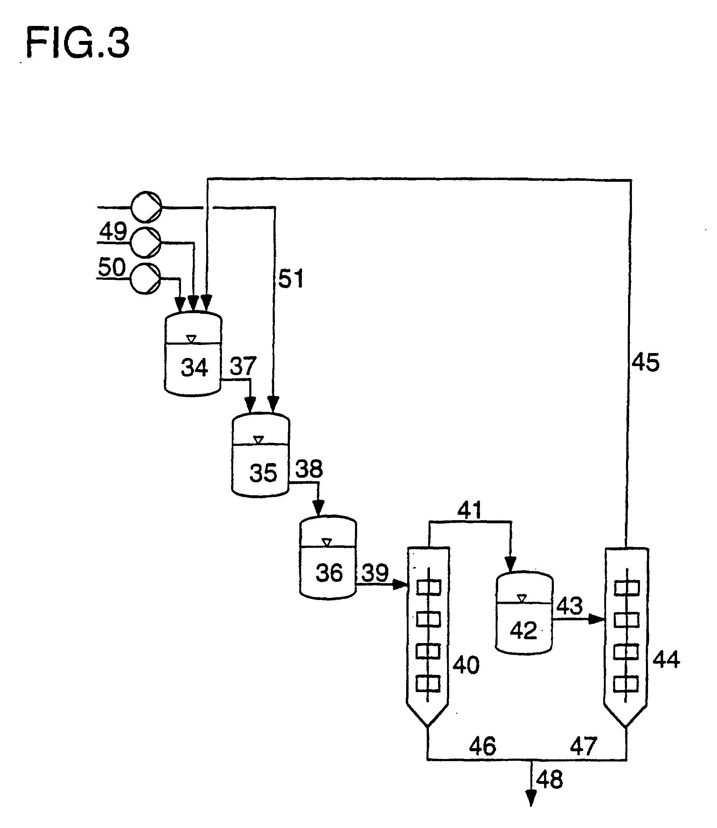

Die folgenden Versuche wurden in einer Laborapparatur, wie sie schematisch in Zeichnung 3 dargestellt ist, durchgeführt. Die Laborapparatur bestand aus einer Kaskade aus 3 beheizbaren, durch die Überlaufrohre 37 und 38 miteinander verbundenen Rührkessel 34, 35 und 36, die ein Fassungsvermögen von jeweils 1000 ml hatten. Der Überlauf des Rührkessels 36 wurde direkt durch das Überlaufrohr 39 in den oberen Teil des Sambay-Verdampfers 40 geleitet und dort destillativ in ein leichtsiedendes Kopfprodukt und ein hochsiedendes Sumpfprodukt aufgetrennt. Das Kopfprodukt wurde über Leitung 41 nach Kondensation (nicht eingezeichnet) dem Nachreaktor 42, einem beheizbaren Rührkessel mit 500 ml Fassungsvermöqen, zugeführt, von dem aus die flüssige Reaktionsmischung über das Überlaufrohr 43 direkt in den oberen Teil eines zweiten Sambay-Verdampfers 44 geleitet wurde. Im Sambay-Verdampfer 44 wurde das so erhaltene Reaktionsgemisch destillativ in ein leichtsiedendes Kopfprodukt und ein hochsiedendes Sumpfprodukt aufgetrennt und das Kopfprodukt über Leitung 45 nach Kondensation (nicht eingezeichnet) in den Rührkessel 34 der Kaskade zurückgeführt. Die Sumpfausträge der Sambay-Verdampfer 40 und 44 wurden über die Leitungen 46 und 47 abgezogen und zusammengeführt und über Leitung 48 in ein Sammelgefäß (nicht eingezeichnet) geleitet. Die so gesammelten Sumpfausträge wurden diskontinuierlich, wie in den Einzelbeispielen beschrieben, weiterverarbeitet. Der Rührkessel 34 wurde außer durch den Rückführstrom von Leitung 45 über die Leitungen 49 und 50 mit FA-Lösung und Amin-Katalysator NR₃ beschickt. Die n-BA-Zufuhr erfolgte über Leitung 51 in den Rührkessel 35.

Beispiele 5 bis 7: Kontinuierliche Arbeitsweise

### Beispiel 5

Die Apparatur gemäß Zeichnung 3 wurde wie in Tabelle 1 beschrieben mit den Edukten beschickt und bei den angegebenen Temperaturen betrieben. Die Angaben für MMB, DMB und FA wurden mittels HPLC-Analyse bestimmt und in Gew.-% notiert.

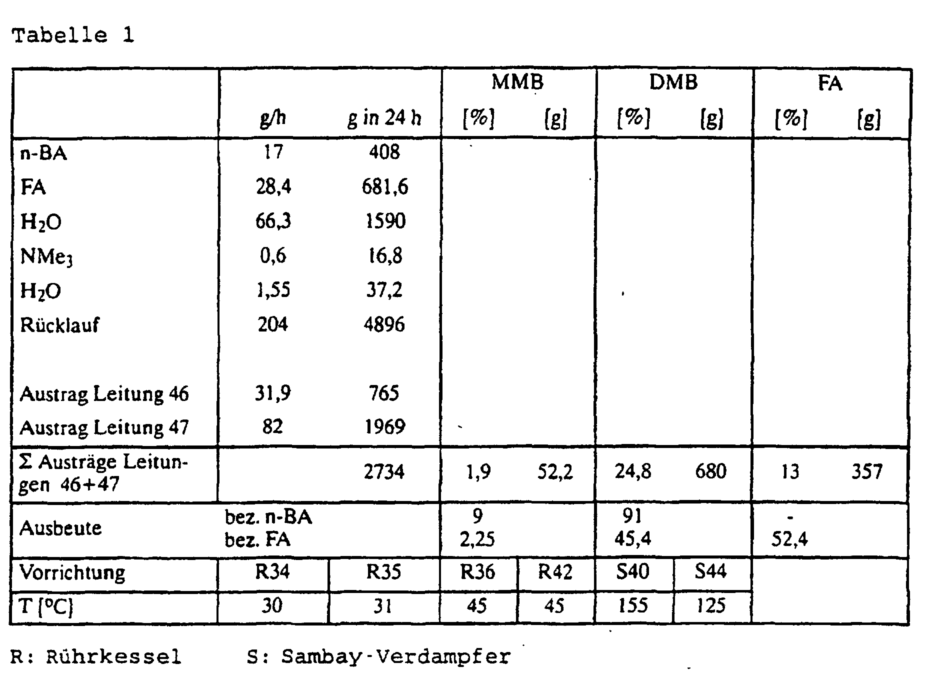

Der Austrag aus Leitung 48 (2734 g) wurde gesammelt und diskontinuierlich bei Atmosphärendruck solange destilliert bis 1025 g Sumpf und 1709 g Destillat erhalten worden sind. Das leichtsiedende Destillat, das 297 g Formaldehyd enthielt, wurde in den Rührkessel 34 zurückgeführt. Diese Menge Formaldehyd kann für die Bilanzierung von der in Tabelle 1 aufgeführten Menge abgezogen werden.

Die so erhaltnen vereinigten Sumpfprodukte wurden wie in Beispiel 1 angegeben hydriert und der Austrag destilliert:

| | MeOH | n-BuOH | Me-BuOH | Diol | TMP | höhere Kondensationsprodukte |
|---|---|---|---|---|---|---|
| Analysenmethode | GC | GC | GC | Auswaage | Auswaage | Auswaage |
| g | 62 | - | 2.5 | 47 | 678 | 14 |
| mol | 2,06 | - | 0,03 | 0,45 | 5,06 | 0,1 |
| Ausbeute (bez. n-BA): 89,3 % Ausbeute (bez. FA inkl. Rückführung): 78.9 % | | | | | | |

### Beispiel 6

Die Apparatur gemäß Zeichnung 3 wurde wie in Tabelle 2 beschrieben mit den Edukten beschickt und bei den angegebenen Temperaturen betrieben.

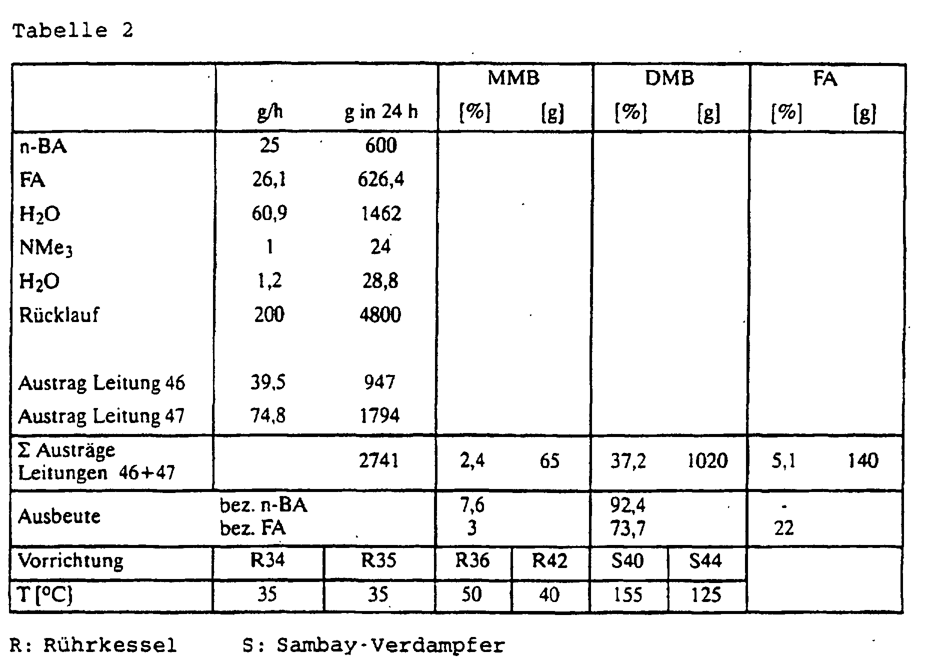

Der Austrag aus Leitung 48 (2741 g) wurde kontinuierlich über einen Sambay-Verdampfer (150°C, Atmosphärendruck) gepumpt (15 ml/h). Es wurden 1506 g Sumpfprodukt und 1235 g Destillat erhalten. Das Destillat, das 105 g Formaldehyd enthielt, wurde in den Rührkessel 34 zurückgeführt. Diese Menge Formaldehyd ist für die Berechnung der Ausbeute von der in Tabelle 2 aufgeführten Menge abzuziehen.

Die so erhaltenen vereinigten Sumpfprodukte wurden wie in Beispiel 1 angegeben hydriert und der Austrag destilliert:

| | MeOH | n-BuOH | Me-BuOH | Diol | TMP | höhere Kondensationsprodukte |
|---|---|---|---|---|---|---|
| Analysenmethode | GC | GC | GC | Auswaage | Auswaage | Auswaage |
| g | 38 | - | 5 | 60 | 1022 | 14 |
| mol | 1,2 | - | 0,057 | 0,58 | 7,6 | 0,1 |
| Ausbeute (bez. n-BA): 91,2 % Ausbeute (bez. FA inkl. Rückführung): 87,4 % | | | | | | |

### Beispiel 7

Die Apparatur gemäß Zeichnung 3 wurde wie in Tabelle 3 beschrieben mit den Edukten beschickt und bei den angegebenen Temperaturen betrieben.

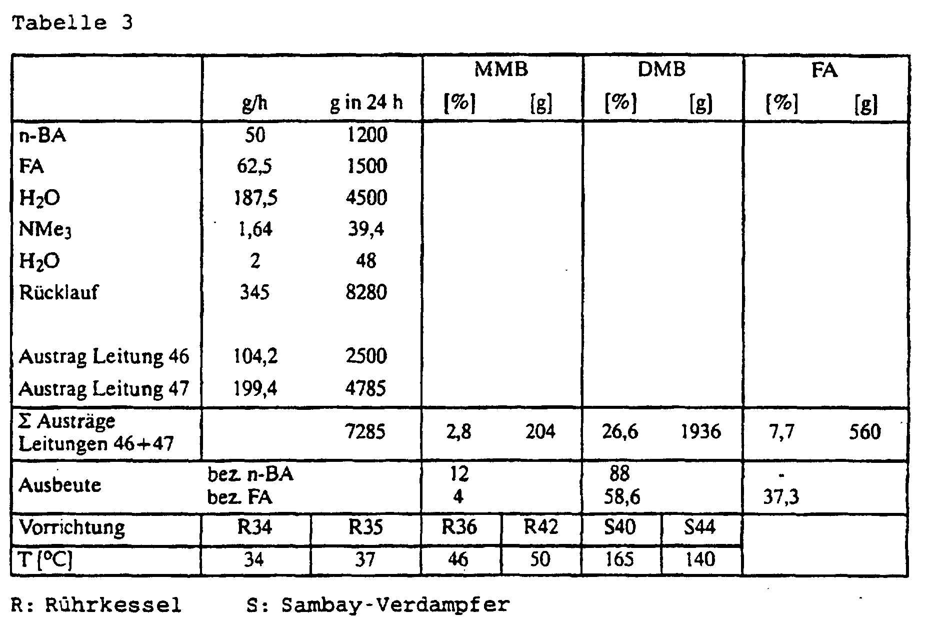

Die gesammelten vereinigten Austräge aus den Leitungen 46 und 47 (7285 g) wurden mit 43 g 45%iger Trimethylaminlösung bei 40°C 3 h gerührt. Dadurch sank der Gehalt an MMB auf 1 %, der von DMB stieg auf 28,7 %. Anschließend wurde die Nachreaktionslösung kontinuierlich über einen Sambay-Verdampfer (150°C, Atmosphärendruck) gepumpt (15 ml/h). Es wurden 4027 g Sumpfprodukt und 3301 g Destillat erhalten. Das Destillat, das 480 g Formaldehyd enthielt, wurde in den Rührkessel 34 zurückgeführt. Diese Menge Formaldehyd kann von der in Tabelle 3 aufgeführten Menge abgezogen werden.

Das so erhaltene Sumpfprodukt wurde wie in Beispiel 1 hydriert und der Austrag destilliert.

| | MeOH | n-BuOH | Me-BuOH | Diol | TMP | höhere Kondensationsprodukte |
|---|---|---|---|---|---|---|
| Analysenmethode | GC | GC | GC | Auswaage | Auswaage | Auswaage |
| g | 89 | - | 17,5 | 97 | 2010 | 67 |
| mol | 2,8 | - | 0,2 | 0,93 | 15 | 0,5 |
| Ausbeute (bez. n-BA): 90 % Ausbeute (bez. FA inkl. Rückführung): 88 % | | | | | | |

### Beispiel 8

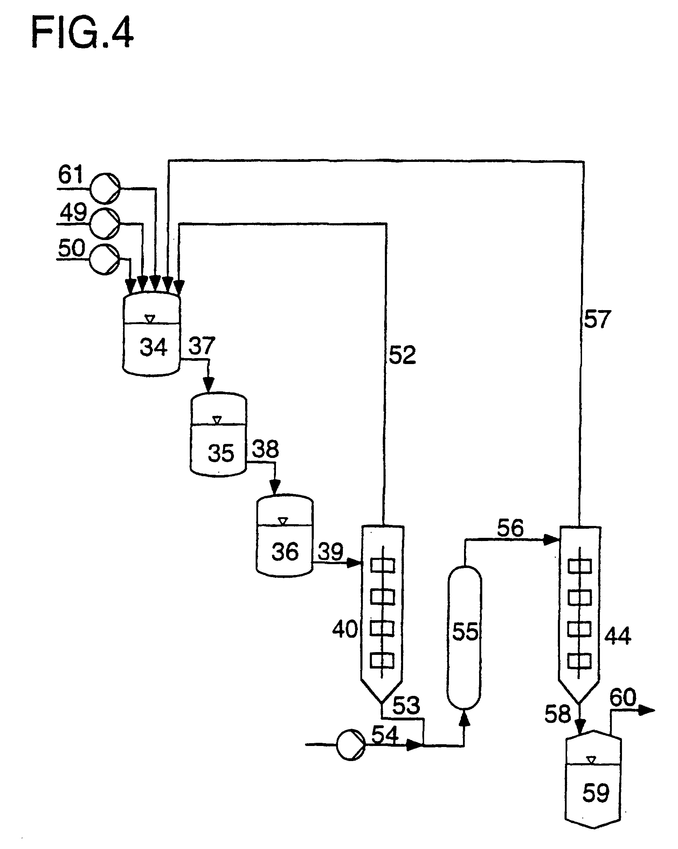

Für die Durchführung dieses Beispiels wurde die für die Beispiele 5 bis 7 verwendete Laborapparatur, wie schematisch in Zeichnung 4 dargestellt, umgebaut. Die umgebaute Laborapparatur bestand aus der bereits beschriebenen (Beispiele 5-7) Kaskade aus den Rührkesseln 34, 35 und 36 und den Überlaufrohren 37, 38 und 39 und dem Sambay-Verdampfer 40. Der Überlauf des Rührkessels 36 wurde direkt durch das Überlaufrohr 39 in den oberen Teil des Sambayverdampfers 40 geleitet und dort destillativ in ein leichtsiedenden Kopfprodukt, das als Komponenten im wesentlichen n-BA, FA, EA, Wasser und Trimethylamin enthielt, und ein hochsiedendes Sumpfprodukte aufgetrennt. Das Kopfprodukt wurde kontinuierlich über Leitung 52 nach Kondensation (nicht eingezeichnet) in den Rührkessel 34 zurückgeführt. Das hochsiedende Sumpfprodukt aus dem Sambay-Verdampfer 40 wurde kontinuierlich über Leitung 53, nach Zugabe frischen Trimethylamin-Katalysator (45%ige wäßrige Lösung) über Leitung 54 dem Nachreaktor 55, einem beheizten mit Füllkörpern (2,5 mm Raschig-Ringe) befüllten Rohrreaktor mit einem Leervolumen von 1000 ml, zugeführt. Der Austrag des Nachreaktors 55 wurde kontinuierlich über Leitung 56 in den oberen Teil des Sambay-Verdampfers 44 gegeben und dort destillativ in ein leichtsiedendes Kopfprodukt, das im wesentlichen aus EA, FA, Trimethylamin und Wasser bestand, und ein hochsiedendes Sumpfprodukt aufgetrennt. Das leichtsiedende Kopfprodukt wurde kontinuierlich über Leitung 57, nach Kondensation (nicht eingezeichnet) in den Rührkessel 34 zurückgeführt, wohingegen das hochsiedende Sumpfprodukt über Leitung 58 im Sammelgefäß 59 gesammelt und von dort über Leitung 60 für die nachfolgende Hydrierung entnommen wurde. Außer mit den Rückführströmen von Leitung 52 und Leitung 57 wurde der Rührkessel 34 über die Leitungen 49 bzw. 61 mit frischer FA-Lösung bzw. n-BA und über Leitung 50 mit frischem Trimethylamin-Katalysator in Form einer 45%igen wäßrigen Lösung kontinuierlich beschickt.

Die zugeführten Mengen an Edukten und Katalysator sind ebenso wie die Mengen der Austräge von Sumpfprodukt aus den Sambay-Verdampfern 40 und 44 und die Zusammensetzung des letztgenannten Austrags in Tabelle 4 aufgelistet. Die Gehalte an MMB, DMB und FA wurden mittels HPLC bestimmt und sind in Gew.-% angegeben. Die in den einzelnen Rührkesseln und Sambay-Verdampfern angewandte Temperatur findet sich ebenfalls in Tabelle 4. Die Destillation in den Sambay-Verdampfern wurde bei Atmosphärendruck ausgeführt.

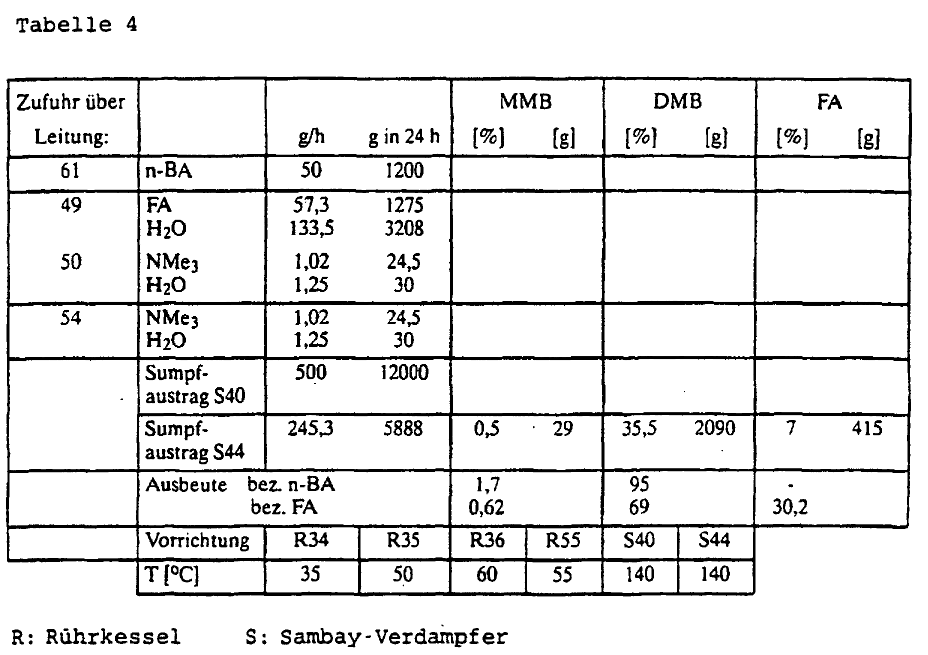

Das Sumpfprodukt aus dem Sammelgefäß 59 wurde, wie in Beispiel 1 beschrieben, hydriert und der Austrag destilliert:

| | MeOH | n-BuOH | Me-BuOH | Diol | TMP | höhere Kondensationsprodukte |
|---|---|---|---|---|---|---|
| Analysenmethode | GC | GC | GC | Auswaage | Auswaage | Auswaage |
| g | 445 | - | 10 | 40 | 2077 | 25 |
| mol | 13,9 | - | 0,11 | 0,4 | 15,5 | 0,19 |
| Ausbeute (bez. n-BA): 93 % Ausbeute (bez. FA): 68 % | | | | | | |

## Patentansprüche

1. Verfahren zur Herstellung von Hydroxymethylalkanalen der Formel I 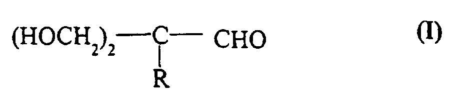 in der R eine weitere Methylolgruppe oder eine Alkylgruppe mit 1 bis 22 C-Atomen oder eine Aryl- oder Aralkylgruppe mit 6 bis 22 C-Atomen bedeutet, durch Kondensation von Aldehyden mit 2 bis 24 C-Atomen mit Formaldehyd unter Verwendung von tertiären Aminen als Katalysator,
**dadurch gekennzeichnet, daß** man die Kondensation stufenweise durchführt, wobei man
a) in einer ersten (Reaktions-)Stufe die Aldehyde mit 2 oder mehr C-Atomen mit der 2- bis 8-fachen molaren Menge Formaldehyd in Gegenwart eines tertiären Amins als Katalysator umsetzt,
b) in einer zweiten (Trenn-)Stufe entweder das Reaktionsgemisch in einen überwiegend die Verbindungen der Formel I enthaltenden Sumpf und einen aus überwiegend nicht umgesetzten oder teilumgesetzten Ausgangsmaterialien bestehenden Destillatstrom trennt, der in die erste Stufe zurückgeführt wird, oder das Reaktionsgemisch aus der ersten Stufe mittels einer Phasenscheidevorrichtung in eine wäßrige und eine organische Phase trennt und die organische Phase in die erste Stufe zurückführt und
c) in einer dritten (Nachreaktions-Destillations-)Stufe den Sumpf der zweiten Stufe oder die in der zweiten Stufe durch Phasenscheidung erhaltene wäßrige Phase einer katalytischen und/oder thermischen Behandlung unterwirft, dabei die unvollständig methylolierte Verbindung der Formel II 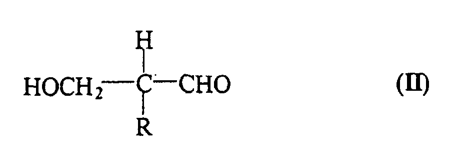 in die entsprechende Verbindung der Formel I und in die entsprechende Methylenverbindung der Formel III 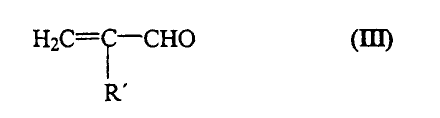 in der R' Wasserstoff ist oder die oben für R genannte Bedeutung hat, überführt, das so erhaltene Reaktionsgemisch destilliert, das Kopfprodukt dieser Destillation, das eine Verbindung der Formel III und nicht umgesetzten Formaldehyd enthält, in die erste Stufe zurückführt,
und als Sumpfprodukt der Destillation die Verbindung der Formel I erhält.

2. Verfahren gemäß Anspruch 1, **dadurch gekennzeichnet, daß** man die rückgeführten Destillate oder die aus Stufe b) rückgeführte organische Phase, sofern sie wesentliche Mengen der Methylenverbindung der Formel III enthalten, einer Vorreaktion mit Formaldehyd und tertiärem Amin unterwirft, ehe sie in Stufe a) mit dem entsprechenden Aldehyd mit 2 bis 24 C-Atomen in Kontakt gebracht werden.

3. Verfahren gemäß Anspruch 1, **dadurch gekennzeichnet, daß** man das Destillat der Stufe b) einer thermischen Nachreaktion unterwirft und erneut destilliert oder gegebenenfalls nach mehrfacher Wiederholung dieser Operation das letzte Destillat in die Stufe a) zurückführt.

4. Verfahren gemäß Anspruch 1, **dadurch gekennzeichnet, daß** Propionaldehyd oder n-Butyraldehyd mit Formaldehyd umgesetzt werden.

5. Verfahren gemäß Anspruch 1, **dadurch gekennzeichnet, daß** Acetaldehyd mit Formaldehyd umgesetzt wird.

6. Verfahren gemäß Anspruch 1, **dadurch gekennzeichnet, daß** man die Umsetzung kontinuierlich durchführt.

7. Verfahren gemäß Anspruch 1, **dadurch gekennzeichnet, daß** man als Katalysator tertiäre Amine in einer solchen Menge verwendet, daß sich im Reaktionsgemisch ein pH-Wert von 5 bis 12 einstellt.

8. Verfahren gemäß Anspruch 1, **dadurch gekennzeichnet, daß** man Trimethylamin als Katalysator verwendet.

9. Verfahren gemäß Anspruch 1, **dadurch gekennzeichnet, daß** man in Stufe c) den gleichen Katalysator wie in Stufe a) verwendet.

10. Verfahren gemäß Anspruch 1, **dadurch gekennzeichnet, daß** man in Stufe a) anstelle eines Aldehyds mit 2 bis 24 C-Atomen oder zusätzlich die entsprechende Verbindung der Formel III als Frischzulauf einspeist.

11. Verfahren gemäß Anspruch 1, **dadurch gekennzeichnet, daß** man in der ersten Stufe a) einen Rohrreaktor oder eine Rührkesselkaskade verwendet.

12. Verfahren gemäß Anspruch 1, **dadurch gekennzeichnet, daß** man in Stufe c) einen Rohrreaktor oder eine Rührkesselkaskade verwendet.

13. Verfahren zur Herstellung von Polymethylolverbindungen der Formel IV
(HOCH₂)₃―C―R (IV)
in der R eine weitere Methylolgruppe oder eine Alkylgruppe mit 1 bis 22 C-Atomen oder eine Aryl- oder Aralkylgruppe mit 6 - 22 C-Atomen bedeutet,
bei dem ein Hydroxymethylalkanal der Formel I 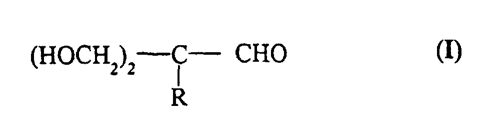 in der R die oben genannte Bedeutung hat und das nach dem Verfahren gemäß einem der Ansprüche 1 bis 12 erhalten wird, in an sich bekannter Weise zu der Polymethylolverbindung der Formel IV hydriert wird.

14. Verfahren nach Anspruch 13 zur Herstellung von Trimethylolethan oder Trimethylolpropan.

15. Verfahren nach Anspruch 13 zur Herstellung von Pentaerythrit.

## Claims

1. A process for preparing hydroxymethylalkanals of the formula I 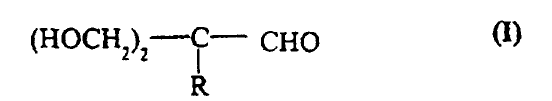 where R is a further methylol group or an alkyl group having from 1 to 22 carbon atoms or an aryl or aralkyl group having from 6 to 22 carbon atoms, by condensation of aldehydes having from 2 to 24 carbon atoms with formaldehyde by use of tertiary amines as a catalyst,
**characterized in that** the condensation is carried out in stages by
a) in a first (reaction) stage reacting the aldehydes having 2 or more carbon atoms with from 2 to 8 times the molar amount of formaldehyde in the presence of a tertiary amine as a catalyst,
b) in a second (separation) stage either separating the reaction mixture into a bottom product, which predominantly contains the compounds of the formula I, and a distillate stream which is composed of predominantly unconverted to partially converted starting materials and which is recycled into the first stage, or separating the reaction mixture from the first stage into an aqueous phase and an organic phase by means of a phase separator and recycling the organic phase into the first stage, and
c) in a third (supplementary reaction/distillation) stage subjecting the bottom product of the second stage or the aqueous phase obtained by phase separation in the second stage to a catalytic and/or thermal treatment to convert the incompletely methylolated compound of the formula II 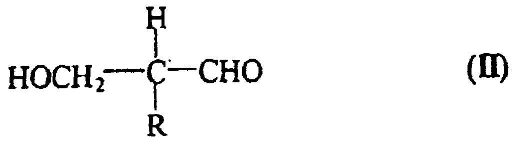 into the corresponding compound of the formula I and into the corresponding methylene compound of the formula III 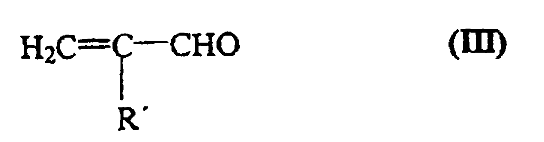 where R' is hydrogen or has the meanings mentioned above for R, distilling the reaction mixture thus obtained, recycling the top product of this distillation, which contains a compound of the formula III and unconverted formaldehyde, into the first stage,
and obtaining the compound of the formula I as a bottom product of the distillation.

2. A process according to claim 1, **characterized in that** the recycled distillates or the organic phase recycled from stage b) are subjected, if they contain significant amounts of the methylene compound of the formula III, to a preliminary reaction with formaldehyde and tertiary amine before they are contacted with the corresponding aldehyde having from 2 to 24 carbon atoms in stage a).

3. A process according to claim 1, **characterized in that** the distillate of step b) is subjected to a thermal, supplementary reaction and redistilled or, if necessary after multiple repetition of this operation, the last distillate is recycled into stage a).

4. A process according to claim 1, **characterized in that** propionaldehyde or n-butyraldehyde are reacted with formaldehyde.

5. A process according to claim 1, **characterized in that** acetaldehyde is reacted with formaldehyde.

6. A process according to claim 1, **characterized in that** the reaction is carried out continuously.

7. A process according to claim 1, **characterized in that** the catalyst used comprises tertiary amines in such an amount that the reaction mixture pH is in the range from 5 to 12.

8. A process according to claim 1, **characterized in that** the catalyst used is trimethylamine.

9. A process according to claim 1, **characterized in that** the same catalyst is used in stage c) as in stage a).

10. A process according to claim 1, **characterized in that** in lieu of or in addition to an aldehyde having from 2 to 24 carbon atoms the corresponding compound of the formula III is fed as a virgin feed into stage a).

11. A process according to claim 1, **characterized in that** the first stage a) utilizes a tubular reactor or a stirred tank battery.

12. A process according to claim 1, **characterized in that** stage c) utilizes a tubular reactor or a stirred tank battery.

13. A process for preparing polymethylol compounds of the formula IV
(HOCH₂)₃―C―R (IV)
where R is a further methylol group or an alkyl group having from 1 to 22 carbon atoms or an aryl or alkyl group having 6-22 carbon atoms,
which comprises hydrogenating a hydroxymethylalkanal of formula I 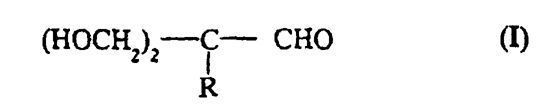 in which R is as defined above and which is obtained by the process of any one of claims 1 to 12, to the polymethylol compound of the formula IV in a conventional manner.

14. A process according to claim 13 for preparing trimethylolethane or trimethylolpropane.

15. A process according to claim 13 for preparing pentaerythritol.

## Revendications

1. Procédé pour la préparation des hydroxyméthylalcanals de formule I 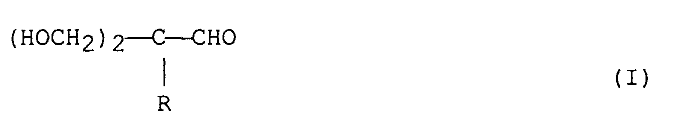 dans laquelle R représente un groupe méthylol supplémentaire ou un groupe alkyle présentant 1 à 22 atomes de C ou un groupe aryle ou aralkyle présentant 6 à 22 atomes de C, au moyen d'une condensation des aldéhydes présentant 2 à 24 atomes de C avec le fomaldéhyde, en mettant en oeuvre des amines tertiaires en tant que catalyseur,
**caractérisé en ce que** l'on réalise la condensation par étapes, procédé dans lequel
a) on met à réagir, dans une première étape (de réaction), les aldéhydes présentant 2 atomes ou plus de C, avec le formaldéhyde en quantité de 2 à 8 fois plus, en présence de l'amine tertiaire en tant que catalyseur,
b) on procède, dans une deuxième étape (de séparation), soit à une séparation du mélange réactionnel afin d'obtenir un produit de fond contenant pour l'essentiel les composés de formule I et un courant de distillat que l'on recycle dans la première étape et qui est constitué de la matière de départ ayant, pour l'essentiel, réagie partiellement ou n'ayant pas réagie, soit à une séparation du mélange réactionnel provenant de la première étape, afin d'obtenir une phase aqueuse et une phase organique, au moyen d'un dispositif de séparation de phases, suivi d'un recyclage de la phase organique dans la première étape, et
c) on soumet à un traitement catalytique et/ou thermique, dans une troisième étape (de distillation et réaction ultérieure), le produit de fond obtenu dans la deuxième étape ou bien la phase aqueuse obtenue dans la deuxième étape au moyen de la séparation des phases, en transformant le composé incomplètement méthylolé de formule II 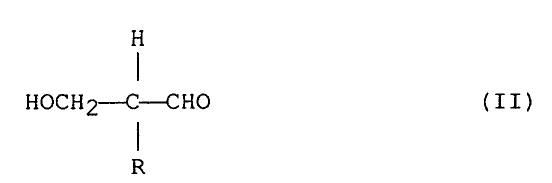 en composé correspondant de formule I et en composé méthylène correspondant de formule III 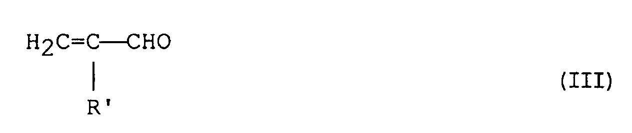 dans laquelle R' représente un atome d'hydrogène ou prend la signification susmentionnée de R, puis on soumet le mélange réactionnel ainsi obtenu à une distillation, on recycle dans la première étape le produit de tête résultant de cette distillation et contenant le composé de formule III et le formaldéhyde qui n'a pas réagi,
et on obtient le composé de formule I en tant que produit de fond de la distillation.

2. Procédé selon la revendication 1, **caractérisé en ce que** l'on soumet à une réaction préalable avec le formaldéhyde et l'amine tertiaire, les distillats recyclés ou la phase organique recyclée qui provient de l'étape b), à condition qu'ils contiennent des quantités intéressantes de composé méthylène de formule III, avant de les mettre en contact, dans l'étape a), avec l'aldéhyde correspondant et présentant 2 à 24 atomes de C.

3. Procédé selon la revendication 1, **caractérisé en ce que** l'on soumet le distillat provenant de l'étape b) à une réaction ultérieure thermique, suivie d'une distillation supplémentaire, ou **en ce que** l'on recycle, éventuellement après avoir répété à plusieurs reprises cette dernière opération, le dernier distillat dans l'étape a).

4. Procédé selon la revendication 1, **caractérisé en ce que** l'on met à réagir le propionaldéhyde ou le n-butyraldéhyde avec le formaldéhyde.

5. Procédé selon la revendication 1, **caractérisé en ce que** l'on met à réagir l'acétaldéhyde avec le formaldéhyde.

6. Procédé selon la revendication 1, **caractérisé en ce que** l'on réalise la réaction de manière continue.

7. Procédé selon la revendication 1, **caractérisé en ce que** l'on met en oeuvre, en tant que catalyseur, l'amine tertiaire en une quantité telle que l'on obtient, dans le mélange réactionnel, un pH compris entre 5 et 12.

8. Procédé selon la revendication 1, **caractérisé en ce que** l'on met en oeuvre la triméthylamine en tant que catalyseur.

9. Procédé selon la revendication 1, **caractérisé en ce que** l'on met en oeuvre, dans l'étape c), le même catalyseur que dans l'étape a).

10. Procédé selon la revendication 1, **caractérisé en ce que** l'on introduit dans l'étape a), à la place ou en supplément de l'aldéhyde présentant 2 à 24 atomes de C, le composé correspondant de formule III en tant que nouveau produit amené.

11. Procédé selon la revendication 1, **caractérisé en ce que** l'on emploie un réacteur tubulaire ou une cascade de cuves malaxeuses dans la première étape a).

12. Procédé selon la revendication 1, **caractérisé en ce que** l'on emploie un réacteur tubulaire ou une cascade de cuves malaxeuses dans l'étape c).

13. Procédé pour la préparation des composés polyméthylol de formule IV :
(HOCH₂)₃―C―R (IV)
dans laquelle R représente un groupe méthylol supplémentaire ou un groupe alkyle présentant 1 à 22 atomes de C ou un groupe aryle ou aralkyle présentant 6 à 22 atomes de C, procédé dans lequel on soumet à une hydrogénation, de manière généralement connue, un hydroxyméthylalcanal de formule I : 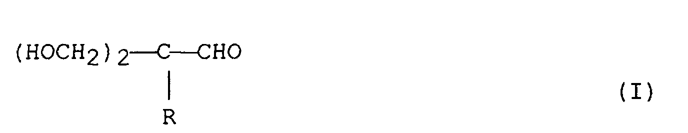 dans laquelle R prend la signification susmentionnée et que l'on obtient selon le procédé selon l'une quelconque des revendications 1 à 12, afin d'obtenir le composé polyméthylol de formule IV.

14. Procédé selon la revendication 13 pour la préparation du triméthyloléthane ou du timéthylolpropane.

15. Procédé selon la revendication 13 pour la préparation du pentaérythrol.
